(19) Europäisches Patentamt
European Patent Office
Office européen des brevets

(11) Veröffentlichungsnummer: **0 391 180**
**A2**

# (12) EUROPÄISCHE PATENTANMELDUNG

(21) Anmeldenummer: **90105614.3**

(22) Anmeldetag: **24.03.90**

(51) Int. Cl.[5]: **C07K 5/06, C07D 233/64, A61K 37/64, C07K 5/08**

Die Bezeichnung der Erfindung wurde geändert (Richtlinien für die Prüfung im EPA, A-III, 7.3).

(30) Priorität: **04.04.89 CH 1266/89**

(43) Veröffentlichungstag der Anmeldung:
**10.10.90 Patentblatt 90/41**

(84) Benannte Vertragsstaaten:
**AT BE CH DE DK ES FR GB GR IT LI LU NL SE**

(71) Anmelder: **F. HOFFMANN-LA ROCHE AG**
**Postfach 3255**
**CH-4002 Basel(CH)**

(72) Erfinder: **Branca, Quirico, Dr.**
**Lenzgasse 2**
**CH-4056 Basel(CH)**
Erfinder: **Edenhofer, Albrecht, Dr.**
**Rudolf Wackernagelstrasse 35**
**CH-4125 Riehen(CH)**
Erfinder: **Märki, Hans Peter, Dr.**
**Seltisbergerstrasse 75**
**CH-4059 Basel(CH)**
Erfinder: **Neidhart, Werner, Dr.**
**Oltmannstrasse 14**
**D-7800 Freiburg im Breisgau(DE)**
Erfinder: **Ramuz, Henri, Prof. Dr.**
**Rheinparkstrasse 3**
**CH-4127 Birsfelden(CH)**
Erfinder: **Wostl, Wolfgang, Dr.**
**Im Strick 2**
**D-7889 Grenzach-Wyhlen(DE)**

(74) Vertreter: **Kellenberger, Marcus, Dr. et al**
**Grenzacherstrasse 124 Postfach 3255**
**CH-4002 Basel(CH)**

## (54) Peptidartige Aminosäurederivate mit reninhemmender Wirkung.

(57) Die Verbindungen der Formel

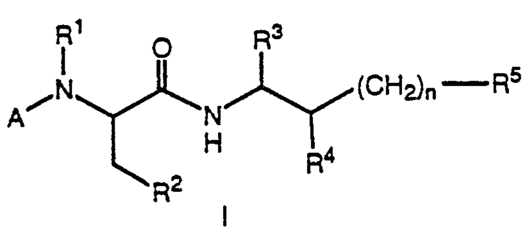

I

worin A, $R^1$, $R^2$, $R^3$, $R^4$, $R^5$ und n die in Anspruch 1 angegebene Bedeutung besitzen,
in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können nach verschiedenen, an sich bekannten Verfahren hergestellt werden.

EP 0 391 180 A2

Xerox Copy Centre

## Peptidartige Aminosäurederivate

Die vorliegende Erfindung betrifft Aminosäurederivate. Im speziellen betrifft sie Aminosäurederivate der allgemeinen Formel

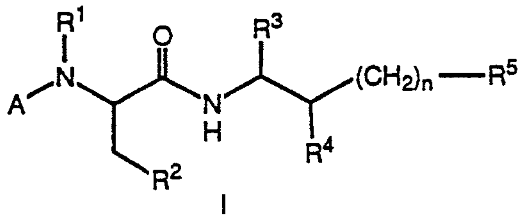

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Hydroxy oder Amino, $R^5$ Alkyl, Haloalkyl, Cycloalkyl, Alkenyl, Aryl oder Heteroaryl, n 0, 1, 2, 3, 4, 5 oder 6 und A eine der Gruppen

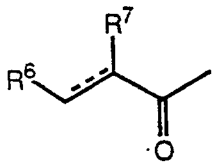 (a) und -Y-Z (b)

bedeuten, worin die gestrichelte Linie eine zusätzliche Bindung bedeuten kann, $R^6$ Phenyl, substituiertes Phenyl, Benzyl oder Naphthyl und $R^7$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl, Y den N-terminal mit Z verbundenen, bivalenten Rest von gegebenenfalls N- und/oder $\alpha$-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, $\alpha$-Naphthylalanin oder Homophenylalanin und Z Wasserstoff oder Acyl bedeuten, mit den Massgaben, dass

(i) falls n 2, 3, 4, 5 oder 6 bedeutet, $R^5$ nicht Alkyl, Cycloalkyl oder Aryl bedeutet,

(ii) falls n 1 und $R^5$ Alkyl bedeuten, das an die $CH_2$-Gruppe gebundene Kohlenstoffatom von Alkyl verzweigt ist,

(iii) falls n 0 und $R^5$ Alkyl bedeuten, das an das den Substituenten $R^4$ tragende Kohlenstoffatom gebundene Kohlenstoffatom von $R^5$ in $\alpha$-Stellung keine Methylengruppe aufweist,

(iv) falls $R^2$ Imidazol-4-yl, $R^4$ Hydroxy und Y Phenylalanin bedeuten, $R^5$ nicht Alkyl bedeutet und

(v) falls $R^6$ Phenyl, Benzyl oder $\alpha$-Naphthyl bedeutet, $R^7$ nicht Alkoxycarbonylamino oder Arylalkoxycarbonylamino bedeutet, in Form von optisch reinen Diastereomeren, Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere

bei der Bekämpfung bzw. Verhütung von Bluthochdruck und Herzinsuffizienz.

Die nachstehenden Definitionen der in der vorliegenden Beschreibung verwendeten allgemeinen Ausdrücke besitzen ihre Gültigkeit unabhängig davon, ob die fraglichen Ausdrücke allein oder in Kombination aufscheinen.

Der in der vorliegenden Beschreibung verwendete Ausdruck "Alkyl" bedeutet geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Isobutoxy, sec.-Butoxy, t-Butoxy und dergleichen. Der Ausdruck "Cycloalkyl" bedeutet gesättigte, cyclische Kohlenwasserstoffreste mit 3-8, vorzugsweise 3-6, Kohlenstoffatomen, wie Cyclopropyl, Cyclobutyl, Cyclopentyl, Cyclohexyl und dergleichen. Der Ausdruck "Heterocycloalkyl" betrifft in ähnlicher Weise gesättigte, 3-8-gliedrige, vorzugsweise 5- oder 6-gliedrige, cyclische Kohlenwasserstoffreste, in welchen eine oder zwei Methylengruppen durch ein oder zwei Sauerstoff- , Schwefel- oder gegebenenfalls durch Alkyl, Phenylalkyl, Alkanoyl oder Alkanoyloxy substituierte Stickstoffatome ersetzt sind, wie Piperidinyl, Pyrazinyl, N-Benzylpyrazinyl, Morpholinyl, N-Methylpiperidinyl, N-Benzylmorpholinyl und dergleichen. Der Ausdruck "Haloalkyl" bedeutet Alkyl, worin ein Wasserstoffatom durch Halogen ersetzt ist. Der Ausdruck "Alkenyl" betrifft geradkettige und verzweigte ungesättigte Kohlenwasserstoffreste mit 2-8, vorzugsweise 2-4, Kohlenstoffatomen, wie Vinyl, Allyl, 2-Butenyl, 3-Butenyl, 3-Pentenyl und dergleichen. Der Ausdruck "Halogen" betrifft die vier Halogene Fluor, Chlor, Brom und Jod. Der Ausdruck "Alkanoyl" bedeutet den Säurerest einer geradkettigen oder verzweigten Alkansäure mit 1-8, vorzugsweise 1-4, Kohlenstoffatomen, wie Formyl, Acetyl, Propionyl, Butyryl, Valeryl, Isovaleryl und dergleichen. Der Ausdruck "Aryl" bezeichnet einen gegebenenfalls durch Alkyl, Alkoxy, Alkanoyloxy, Amino, Alkylamino, Dialkylamino, Alkylcarbonylamino, Hydroxyl, Halogen, Trifluormethyl oder Nitro ein- oder mehrfach substituierten mono- oder bicyclischen aromatischen Kohlenwasserstoffrest mit 6-14 Kohlenstoff atomen, wie Phenyl, $\alpha$- oder $\beta$-Naphthyl, Indenyl, Anthryl oder Phenanthryl und dergleichen. Der Ausdruck "Arylalkyl" bezeichnet geradkettige oder verzweigte Alkylgruppen, worin ein oder mehrere Wasserstoffatome durch Arylgruppen ersetzt sind, wie Benzyl, Diphenylmethyl, Trityl, $\alpha$- oder $\beta$-Naphthylmethyl, 2-Phenyläthyl, 3-Phenyl-2-propyl, 4-Phenyl-3-butyl, 2-($\alpha$- oder $\beta$-Naphthyl)äthyl, 3-$\alpha$-Naphthyl-2-propyl, 4-$\alpha$-Naphthyl-3-butyl und dergleichen, wobei der aromatische Rest jeweils wie oben angegeben ein- oder mehrfach substituiert sein kann. Der Ausdruck "substituiertes Phenyl" bezeichnet gegebenenfalls durch Alkyl, Alkoxy, Alkoxyalkoxy, Alkanoyl, Alkanoyloxy, Hydroxy, Halogen oder Trifluormethyl ein- oder mehrfach substituiertes Phenyl, wie 4-Hydroxyphenyl, 4-Methoxyphenyl, 4-Methylphenyl, 4-Chlorphenyl, 4-Aethoxyäthoxyphenyl und dergleichen. Der Ausdruck "Heteroaryl" bezeichnet einen gegebenenfalls an einem Stickstoffatom durch Alkyl, Phenyl oder Phenylalkyl und/oder an einem oder mehreren Kohlenstoffatomen durch Alkyl, Phenyl, Phenylalkyl, Halogen, Hydroxy, Alkoxy, Phenylalkoxy oder Oxo substituierten und teilweise gesättigten mono- oder bicyclischen aromatischen Kohlenwasserstoffreste, in welchem ein oder mehrere Kohlenstoffatome durch ein bis zwei Stickstoffatome und/oder ein Sauerstoff- oder Schwefelatom ersetzt sind, wie Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl oder Chinoxalinyl, z.B. 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl und dergleichen.

Der Ausdruck "substituiertes Amino" bedeutet eine durch Alkyl, Arylalkyl, Alkanoyl, Alkoxycarbonyl oder Arylalkoxycarbonyl mono- oder di-substituierte oder eine gegebenenfalls durch ein Sauerstoff-, Schwefel- oder gegebenenfalls Alkyl-, Phenylalkyl-, Alkanoyl- oder Alkanoyloxy-substituiertes Stickstoffatom unterbrochene $C_3$-$C_6$-Alkylen disubstituierte Aminogruppe. Der Ausdruck "Acyl" betrifft die Acylgruppe einer Carbonsäure, eines Halbesters der Kohlensäure, einer gegebenenfalls N-substituierten Carbamin- oder Thiocarbaminsäure, eines gegebenenfalls N-substituierten Oxalamids, einer Sulfonsäure oder einer gegebenenfalls N-substituierten Amidosulfonsäure, insbesondere solche mit den Teilformeln $R^b$-CO-, $R^a$-O-CO-, $(R^b)(R^b)$N-CO-, $(R^b)(R^b)$N-CS-, $(R^b)(R^b)$N-CO-CO-, $R^b$-$SO_2$-, oder $(R^b)(R^b)$N-$SO_2$-, worin $R^a$ einen unsubstituierten oder substituierten, gesättigten oder ungesättigten, gegebenenfalls mit Amino, Monoalkylamino, Dialkylamino, Alkanoylamino oder Alkanoyloxyamino funktionalisierten aliphatischen, cycloaliphatischen, cycloaliphatisch-aliphatischen Kohlenwasserstoffrest mit bis zu 18, vorzugsweise 10, Kohlenstoffatomen, einen unsubstituierten oder substituierten aromatischen, heteroaromatischen, aromatisch-aliphatischen oder heteroaromatisch-aliphatischen Kohlenwasserstoffrest mit bis zu 18, vorzugsweise 10, Kohlenstoffatomen oder einen unsubstituierten oder substituierten, gesättigten 5- oder 6-gliedrigen heterocyclischen Rest

bedeutet und $R^b$ Wasserstoff bedeutet oder die Bedeutung von $R^a$ besitzt. Der Ausdruck "Acyl" betrifft auch den einwertigen, über die Carboxylgruppe gebundenen Rest einer gegebenenfalls acylierten Aminosäure oder eines gegebenenfalls acylierten Dipeptids.

Ein unsubstituierter oder substituierter, gesättigter oder ungesättigter, aliphatischer, cycloaliphatischer oder cycloaliphatisch-aliphatischer Kohlenwasserstoffrest $R^a$ oder $R^b$ ist beispielsweise unsubstituiertes oder substituiertes Alkyl, Alkenyl, Alkinyl, Mono-, Bi- oder Tricycloalkyl, Monocycloalkenyl, Bicycloalkenyl, Cycloalkylalkyl, Cycloalkylalkenyl oder Cycloalkenylalkyl. "Substituiertes Alkyl" bedeutet einen Alkylrest, in welchem ein oder mehrere Wasserstoffatome durch Hydroxy, Alkoxy, Aryloxy, Alkanoyloxy, Halogen, Hydroxysulfonyloxy, Carboxy, Alkoxycarbonyl, Carbamoyl, Alkylcarbamoyl, Dialkylcarbamoyl, Cyan, Phosphono, verestertes Phosphono, Amino oder Oxo substituiert sein können, wobei die Substituenten nur dann in 1-Stellung des Alkylrestes stehen, wenn dieser in der Teilformel $R^b$-CO- an die Carbonylgruppe gebunden ist.

Beispiele von substituiertem Alkyl sind 2-Hydroxyäthyl, Methoxymethyl, 2-Methoxyäthyl, Phenoxymethyl, α- oder β-Naphthoxymethyl, Acetoxymethyl, 2-Acetoxyäthyl, Chlormethyl, Brommethyl, 2-Chlor- oder 2-Bromäthyl, Hydroxysulfonyloxymethyl, 2-Hydroxysulfonyloxyäthyl, Carboxymethyl, 2-Carboxyäthyl, Methoxycarbonylmethyl, 2-Methoxycarbonyläthyl, Aethoxycarbonylmethyl, 2-Aethoxycarbonyläthyl, Carbamoylmethyl, 2-Carbamoyläthyl, Methylcarbamoylmethyl, Dimethylcarbamoylmethyl, Cyanomethyl, 2-Cyanoäthyl, 2-Oxopropyl, 2-Oxobutyl, Hydroxycarboxymethyl, 1-Hydroxy-2-carboxyäthyl, Hydroxyäthoxycarbonyläthyl, Hydroxymethoxycarbonyläthyl, Acetoxymethoxycarbonylmethyl, 1,2-Dihydroxy-2-carboxyäthyl, 1,2-Dihydroxy-2-äthoxycarbonyläthyl, 1,2-Dihydroxy-2-methoxycarbonyläthyl, 1,2-Diacetoxy-2-äthoxycarbonyläthyl, 1,2-Diacetoxy-2-methoxycarbonyläthyl, 1-α-Naphthoxy-3-carboxypropyl, 1-α-Naphthoxy-2-äthoxycarbonyläthyl, 1-α-Naphthoxy-3-t-butoxycarbonylpropyl, 1-α-Naphthoxy-2-benzyloxycarbonyläthyl, 1-α-Naphthoxy-3-carbamoylpropyl, α-Naphthoxycyanomethyl, 1-α-Naphthoxy-3-cyanopropyl, 1-α-Naphthoxy-4-dimethylaminobutyl oder 1-α-Naphthoxy-3-oxobutyl.

Der Ausdruck "Alkenyl" betrifft einen ungesättigten Kohlenwasserstoffrest wie er weiter oben definiert worden ist, wobei die Doppelbindung nur dann in 1-Stellung des Alkenylrestes stehen kann, wenn dieser in der Teilformel $R^b$-CO- an die Carbonylgruppe gebunden ist. Die Alkenylreste können durch die gleichen Substituenten substituiert sein wie die Alkylreste.

Der Ausdruck "Alkinyl" betrifft Kohlenwasserstoffreste mit 2-8, vorzugsweise 2-4, Kohlenstoffatomen, welche eine Dreifachbindung enthalten, wie Aethinyl, 1-Propinyl oder 2-Propinyl. Der Ausdruck "Bicycloalkyl" betrifft bicyclische gesättigte Kohlenwasserstoffreste mit 5-10, vorzugsweise 6-9, Kohlenstoffatomen, wie Bicyclo[3.1.0]hex-1-yl, Bicyclo[3.1.0]hex-2-yl, Bicyclo[3.1.0]hex-3-yl, Bicyclo[4.1.0]hept-1-yl, Bicyclo[4.1.0]hept-4-yl, Bicyclo[2.2.1]hept-2-yl, Bicyclo[3.2.1]oct-2-yl, Bicyclo[3.3.0]oct-3-yl, Bicyclo[3.3.1]-non-9-yl, α- oder β-Decahydronaphthyl und dergleichen.

Der Ausdruck "Tricycloalkyl" betrifft einen tricyclischen gesättigten Kohlenwasserstoffrest mit 8-10 Kohlenstoffatomen, wie 1-Adamantyl.

Der Ausdruck "Cycloalkenyl" betrifft einen ungesättigten cyclischen Kohlenwasserstoffrest mit 3-8, vorzugsweise 3-6, Kohlenstoffatomen, wie 1-Cyclohexenyl, 1,4-Cyclohexadienyl und dergleichen.

Der Ausdruck "Bicycloalkenyl" betrifft einen bicyclischen ungesättigten Kohlenwasserstoffrest mit 5-10, vorzugsweise 7-10, Kohlenstoffatomen, wie 5-Norbornen-2-yl, Bicyclo[2.2.2]octen-2-yl, Hexahydro-4,7-methanoind-1-en-6-yl und dergleichen.

Beispiele für Cycloalkylalkyl sind Cyclopropylmethyl, Cyclobutylmethyl, Cyclopentylmethyl, Cyclohexylmethyl und dergleichen. Als Beispiele für Cycloalkylalkenyl können Cyclohexylvinyl und Cyclohexylallyl und dergleichen genannt werden. Beispiele für Cycloalkenylalkyl sind 1-Cyclohexenylmethyl, 1,4-Cyclohexadienylmethyl und dergleichen.

Die genannten cycloaliphatischen und cycloaliphatisch-aliphatischen Reste können durch die gleichen Substituenten wie Alkyl substituiert sein.

Ein gegebenenfalls substituierter aromatischer oder aromatisch-aliphatischer Kohlenwasserstoffrest ist beispielsweise unsubstituiertes oder substituiertes Aryl, Arylalkyl oder Arylalkenyl. Beispiele für Arylalkenyl sind Styryl, 3-Phenylallyl, 2-(α-Naphthyl)vinyl, 2-(β-Naphthyl)vinyl und dergleichen.

In einem heteroaromatischen oder heteroaromatisch-aliphatischen Kohlenwasserstoffrest ist der Heterocyclus mono-, bi- oder tricyclisch und enthält ein bis zwei Stickstoffatome und/oder ein Sauerstoff- oder Schwefelatom und ist mit einem seiner Ring-Kohlenstoffatome mit der Gruppe -CO-, -O-CO-, >N-CO-, >N-CS-, >N-CO-CO-, $-SO_2$ oder $>N-SO_2$-verknüpft. Beispiele solcher heteroaromatischer Kohlenwasserstoffreste sind Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanneliertes, cyclopenta-, cyclohexa- oder cyclohepta-anneliertes Derivat dieser Reste. Der heteroaromatische Rest kann an einem Stickstoffatom durch Alkyl, Phenyl oder Phenylalkyl, z.B. Benzyl und/oder an einem oder mehreren Kohlenstoffatomen

durch Alkyl, Phenyl, Phenylalkyl, Halogen, Hydroxy, Alkoxy, Phenylalkoxy oder Oxo substituiert und teilweise gesättigt sein. Beispiele solcher heteroaromatischer Reste sind 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl und dergleichen.

Beispiele heteroaromatisch-aliphatischer Kohlenwasserstoffreste sind 2- oder 3-Pyrrolylmethyl, 2-, 3- oder 4-Pyridylmethyl, 2-(2-, 3- oder 4-Pyridyl)äthyl, 4-Imidazolylmethyl, 2-(4-Imidazolyl)äthyl, 2-Indolylmethyl, 3-Indolylmethyl, 2-(3-Indolyl)äthyl, 2-Chinolylmethyl und dergleichen.

Ein gesättigter 5- oder 6-gliedriger heterocyclischer Rest hat mindestens ein Kohlenstoffatom, 1-3 Stickstoffatome und gegebenenfalls ein Sauerstoff- oder Schwefelatom als Ringglieder und ist mit einem seiner Ringkohlenstoffatome mit der Gruppe -CO- bzw. -O-CO-, >N-CO-, >N-CS-, >N-CO-CO-, $-SO_2-$ oder $>N-SO_2-$ verknüpft. Der Heterocyclus kann an einem seiner Kohlenstoffatome oder an einem Ringstickstoffatom durch Alkyl, z.B. Methyl oder Aethyl, Phenyl oder Phenylalkyl, z.B. Benzyl, oder an einem seiner Kohlenstoffatome durch Hydroxy oder Oxo substituiert und/oder an zwei benachbarten Kohlenstoffatomen benzanneliert sein. Beispiele solcher Reste sind Pyrrolidin-3-yl, 4-Hydroxypyrrolidin-2-yl, 5-Oxopyrrolidin-2-yl, Piperidin-2-yl, Piperidin-3-yl, 1-Methylpiperidin-2-yl, 1-Methylpiperidin-3-yl, 1-Methylpiperidin-4-yl, Morpholin-2-yl, Morpholin-3-yl, Thiomorpholin-2-yl, Thiomorpholin-3-yl, 1,4-Dimethylpiperazin-2-yl, 2-Indolinyl, 3-Indolinyl, 1,2,3,4-Tetrahydrochinol-2-, -3- oder -4-yl, 1,2,3,4-Tetrahydroisochinol-1-, -3-oder -4-yl, 1-Oxo-1,2,3,4-tetrahydroisochinol-3-yl und dergleichen.

Als Rest einer über die Carboxylgruppe gebundenen Aminosäure kommen natürliche α-Aminosäuren mit der L-Konfiguration, Homologe solcher Aminosäuren, z.B. worin die Aminosäurenseitenkette um eine oder zwei Methylengruppen verlängert oder verkürzt ist und/oder eine Methylgruppe durch Wasserstoff ersetzt ist, substituierte aromatische α-Aminosäuren, z.B. substituiertes Phenylalanin oder Phenylglycin, worin der Substituent Alkyl, z.B. Methyl, Halogen, z.B. Fluor, Chlor, Brom oder Jod, Hydroxy, Alkoxy, z.B. Methoxy, Alkanoyloxy, z.B. Acetoxy, Amino, Alkylamino, z.B. Methylamino, Dialkylamino, z.B. Dimethylamino, Alkanoylamino, z.B. Acetylamino oder Pivaloylamino, Alkoxycarbonylamino, z.B. t-Butoxycarbonylamino, Arylmethoxycarbonylamino, z.B. Benzyloxycarbonylamino und/oder Nitro sein kann und ein- oder mehrfach vorkommt, benzanneliertes Phenylalanin oder Phenylglycin, wie α-Naphthylalanin, oder hydriertes Phenylalanin oder Phenylglycin, wie Cyclohexylalanin oder Cyclohexylglycin, eine 5- oder 6-gliedrige cyclische benzannelierte α-Aminosäure, z.B. Indolin-2-carbonsäure oder 1,2,3,4-Tetrahydroisochinolin-3-carbonsäure, eine natürliche oder homologe α-Aminosäure, in der eine Carboxygruppe in der Seitenkette in veresterter oder amidierter Form vorliegt, z.B. als Alkylestergruppe, wie Methoxycarbonyl oder t-Butoxycarbonyl, oder als Carbamoyl-, Alkylcarbamoyl, wie Methylcarbamoyl, oder als Dialkylcarbamoylgruppe, wie Dimethylcarbamoyl, in der eine Aminogruppe der Seitenkette in acylierter Form vorliegt, z.B. als Alkanyolamino, wie Acetylamino oder Pivaloylamino, als Alkoxycarbonylamino-, wie t-Butoxycarbonylamino, oder als Arylmethoxycarbonylaminogruppe, wie Benzyloxycarbonylamino, oder in der eine Hydroxygruppe der Seitenkette in verätherter oder veresterter Form vorliegt, z.B. als Alkoxygruppe, wie Methoxy, als Arylalkoxygruppe, wie Benzyloxy, oder als nieder Alkanoyloxygruppe, wie Acetoxy, oder Epimere solcher Aminosäuren, d.h. mit der unnatürlichen D-Konfiguration. Beispiele solcher Aminosäuren sind Glycin, Alanin, Valin, Norvalin, Leucin, Isoleucin, Norleucin, Serin, Homoserin, Threonin, Methionin, Cystein, Prolin, Trans-3- und Trans-4-hydroxyprolin, Phenylalanin, Tyrosin, 4-Nitrophenylalanin, 4-Aminophenylalanin, 4-Chlorphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutaminsäuremono-t-butylester, Glutamin, N-Dimethylglutamin, Histidin, Arginin, Lysin, N-t-Butoxycarbonyllysin, δ-Hydroxylysin, Ornithin, N-Pivaloylornithin, α,γ-Diaminobuttersäure oder α,β-Diaminopropionsäure und dergleichen. Der über die Carboxylgruppe gebundene Rest der Aminosäure kann N-terminal zur Erhöhung der Stabilität der Verbindung der Formel I gegen enzymatischen Abbau durch Alkyl, z.B. Methyl oder Aethyl, substituiert sein.

Der über die Carboxylgruppe gebundene Rest eines Dipeptids besteht aus zwei der oben erwähnten Aminosäuren.

Der Ausdruck "acylierte Aminosäure" bzw. "acyliertes Dipeptid" betrifft eine der oben erwähnten Aminosäuren bzw. ein Dipeptid aus zwei der oben erwähnten Aminosäuren, welche bzw. welches N-terminal durch den Acylrest einer Carbonsäure, eines Halbesters der Kohlensäure, einer gegebenenfalls N-substituierten Carbamin- oder Thiocarbaminsäure, eines gegebenenfalls N-substituierten Oxalamids, einer Sulfonsäure oder einer gegebenenfalls N-substituierten Amidosulfonsäure substituiert ist.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugt sind solche Verbindungen der Formel I, worin $R^1$ Wasserstoff bedeutet. $R^2$ bedeutet vorzugsweise Imidazol-2-yl, Imidazol-4-yl oder Thiazol-4-yl, besonders bevorzugt Imidazol-4-yl. Weiter sind solche Verbindungen der Formel I bevorzugt, worin $R^3$ Cyclohexylmethyl bedeutet. $R^4$ bedeutet vorzugsweise Hydroxy. Bevorzugt sind auch solche Verbindungen der Formel I, worin $R^5$ Alkyl, vorzugsweise 3-Pentyl, Haloalkyl, vorzugsweise Fluoralkyl, Cycloalkyl, vorzugsweise Cyclohexyl, oder Alkenyl, vorzugsweise 3-Pentenyl, bedeutet. Die bevorzugte Bedeutung von n ist 0 oder 1, besonders bevorzugt 1. Ebenfalls bevorzugt sind die Verbindungen der Formel I, worin A die Gruppe (a) bedeutet. $R^6$ bedeutet vorzugsweise Phenyl oder substituiertes Phenyl, besonders bevorzugt Phenyl. Die bevorzugte Bedeutung von $R^7$ ist Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, vorzugsweise Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl. Falls A die Gruppe (b) bedeutet, so sind diejenigen Verbindungen der Formel I bevorzugt, worin Y den N-terminal mit Z verbundenen, bivalenten Rest von Phenylalanin bedeutet. Z bedeutet vorzugsweise die Gruppe $R^a$-O-CO- oder den Rest einer durch diese Gruppe acylierten $\alpha$-Aminosäure, vorzugsweise Prolin, worin $R^a$ einen gegebenenfalls substituierten, gesättigten aliphatischen Kohlenwasserstoffrest mit bis zu 10 Kohlenstoffatomen oder einen gegebenenfalls substituierten heteraromatischen Kohlenwasserstoffrest mit bis zu 18 Kohlenstoffatomen, ganz besonders bevorzugt einen gesättigten, aliphatischen Kohlenwasserstoffrest mit bis zu 6 Kohlenstoffatomen, bedeutet.

Aus dem obigen folgt, dass solche Verbindungen der Formel I ganz besonders bevorzugt sind, worin $R^1$ Wasserstoff, $R^2$ Imidazol-4-yl, $R^3$ Cyclohexylmethyl, $R^4$ Hydroxy, $R^5$ 3-Pentyl, Fluoralkyl, Cyclohexyl oder 3-Pentenyl, n 1, $R^6$ Phenyl, $R^7$ $C_1$-$C_4$-Alkylcarbonylmethyl oder $C_1$-$C_4$-Alkylsulfonylmethyl, Y den N-terminal mit Z verbunden, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen bedeutet.

Ganz speziell bevorzugte Verbindungen der Formel I sind:
t-Butyl (R)-2-[[(S)-$\alpha$-[[(S)-1-[[(1S,2S,4R)-1-(cyclohexylmethyl)-2-hydroxy-4-äthyl-5-hexenyl]carbamoyl]-2-imidazol-4-yläthyl]carbamoyl]phenäthyl]carbamoyl]-1-pyrrolidincarboxylat,
t-Butyl (R)-2-[[(S)-$\alpha$-[[(S)-1-[[(1S,2S)-1-(cyclohexylmethyl)-2-hydroxy-4-äthylhexyl]carbamoyl]-2-imidazol-4-yläthyl]carbamoyl]phenäthyl]carbamoyl]-1-pyrrolidincarboxylat,
(S)-N-[(1S,2S,4R)-1-(Cyclohexylmethyl)-4-äthyl-2-hydroxy-5-hexenyl]-$\alpha$-[(R)-$\alpha$-(3,3-dimethyl-2-oxobutyl)-hydrocinnamamido]imidazol-4-propionamid,
(S)-$\alpha$-[(S)-$\alpha$-[(t-Butylsulfonyl)methyl]hydrocinnamamido]-N-[(1S,2S,4R)-1-(cyclohexylmethyl)-4-äthyl-2-hydroxy-5-hexenyl]imidazol-5-propionamid,
t-Butyl [(S)-$\alpha$-[[(S)-1-[[(1S,2R oder 2S)-3-cyclohexyl-1-(cyclohexylmethyl)-2-hydroxypropyl]carbamoyl]-2-imidazol-4-yläthyl]carbamoyl]phenäthyl]carbamat,
(2R oder S,3S)-3-(Boc-D-Pro-Phe-His-NH)-1,4-dicyclohexyl-2-butanol und
(S)-N-[(1S,2S)-1-(Cyclohexylmethyl)-4-äthyl-4-fluor-2-hydroxyhexyl]-$\alpha$-[(R)-$\alpha$-(3,3-dimethyl-2-oxobutyl)-hydrocinnamamido]imidazol-4-propionamid.

Die Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man

a) zur Herstellung einer Verbindung der Formel I, worin $R^4$ Hydroxy bedeutet und die übrigen Symbole die oben angegebene Bedeutung besitzen, eine Verbindung der allgemeinen Formel

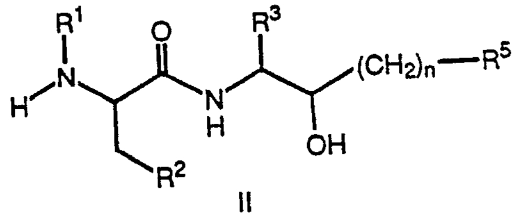

II

worin $R^1$, $R^2$, $R^3$, $R^5$ und n die oben angegebene Bedeutung besitzen, mit einem die Gruppe

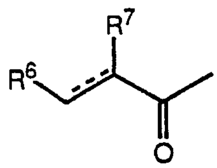

(a) oder -Y-Z (b)

worin $R^6$, $R^7$, Y, Z und die gestrichelte Linie die oben angegebene Bedeutung besitzen, abgebenden Acylierungsmittel umsetzt, oder

b) zur Herstellung einer Verbindung der Formel I, worin $R^4$ Hydroxy bedeutet und die übrigen Symbole die oben angegebene Bedeutung besitzen, eine Verbindung der allgemeinen Formel

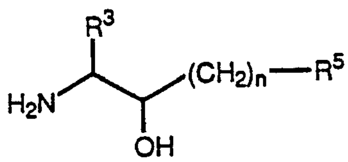

III

worin $R^3$, $R^5$ und n die oben angegebene Bedeutung besitzen, mit einer Verbindung der allgemeinen Formel

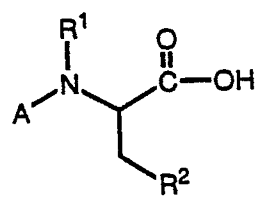

IV

worin $R^1$, $R^2$ und A die oben angegebene Bedeutung besitzen, oder einem aktivierten Derivat davon umsetzt, oder

c) zur Herstellung einer Verbindung der Formel I, worin A die Gruppe (b), Z den einwertigen, über die Carboxylgruppe gebundenen Rest einer gegebenenfalls acylierten Aminosäure oder eines gegebenenfalls acylierten Dipeptids und $R^4$ Hydroxy bedeuten und die übrigen Symbole die oben angegebene Bedeutung besitzen, eine Verbindung der Formel I, worin Z Wasserstoff bedeutet und die übrigen Symbole die oben angegebene Bedeutung besitzen, mit einer gegebenenfalls acylierten Aminosäure oder einem gegebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeuten, aus einer entsprechenden Verbindung der Formel I, worin A eine N-geschützte Aminogruppe enthält, und/oder aus einer Verbindung

der allgemeinen Formel

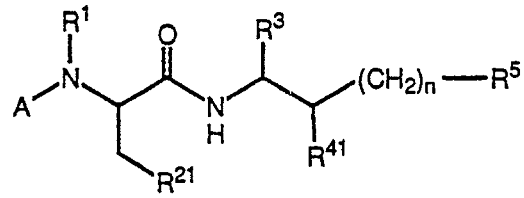

V

worin $R^{41}$ Hydroxy oder N-geschütztes Amino und $R^{21}$ Aethyl, Propyl, Isopropyl, Thiazol-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl bedeuten und die übrigen Symbole die oben angegebene Bedeutung besitzen, mit der Massgabe, dass mindestens einer von $R^{41}$ und $R^{21}$ eine N-Schutzgruppe enthält, die N-Schutzgruppe(n) abspaltet, und

e) erwünschtenfalls ein Gemisch diastereomerer Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0°C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Falls es sich beim Acylierungsmittel um ein Peptid handelt, erfolgt die Reaktion unter in der Peptidchemie üblichen Reaktionsbedingungen, d.h. vorzugsweise in Gegenwart eines Kondensationsmittels wie HBTU (O-Benzotriazolyl-N,N,N′,N′-tetramethyluronium-hexafluorophosphat), BOP (Benzotriazol-1-yloxy-bis-(dimethylamino)phosphonium-hexafluorophosphat), BOPC (Bis(2-oxo-2-oxozolidinyl)phosphinchlorid), HOBT (N-Hydroxybenzotriazol), DBU (1,8-Diazabicyclo[5,4,0]undec-7-en), DCC (Dicyclohexylcarbodiimid), EDC (N-Aethyl-N′(3-dimethylaminopropyl)carbodiimid-hydrochlorid), Hünigbase (Aethyldiisopropylamin), und dergleichen. Die Reaktion wird zweckmässigerweise in einem unter den Reaktionsbedingungen inerten organischen Lösungsmittel oder Lösungsmittelgemisch bei einer Temperatur zwischen etwa 0° und 50°C, vorzugsweise bei etwa Raumtemperatur durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel III mit einer Verbindung der Formel IV erfolgt ebenfalls nach an sich in der Peptid-Chemie bekannten Methoden, d.h. unter den gleichen Bedingungen wie oben für die Umsetzung einer Verbindung der Formel II mit einem Peptid angegeben wurde. Beispiele geeigneter aktivierter Derivate einer Verbindung der Formel IV sind Säurehalogenide, Säureanhydride, gemischte Anhydride, Ester, gemischte Ester, und dergleichen.

Die Umsetzung einer Verbindung der Formel I, worin Z Wasserstoff bedeutet, mit einer gegebenenfalls acylierten Aminosäure oder einem gegebenenfalls acylierten Dipeptid gemäss Verfahrensvariante c) erfolgt ebenfalls nach an sich in der Peptid-Chemie bekannten Methoden, d.h. unter den oben für die Umsetzung einer Verbindung der Formel II mit einem Peptid angegebenen Bedingungen.

Die Abspaltung der N-Schutzgruppe(n) gemäss Verfahrensvariante d) erfolgt ebenfalls nach an sich bekannten Methoden in Abhängigkeit von der Art der abzuspaltenden N-Schutzgruppe. Die Abspaltung erfolgt jedoch zweckmässig durch saure oder basische Hydrolyse. Für die saure Hydrolyse verwendet man vorteilhafter Weise eine Lösung einer Mineralsäure, wie Chlorwasserstoffsäure, Bromwasserstoffsäure, Trifluoressigsäure, Schwefelsäure, Phosphorsäure und dergleichen, in einem inerten Lösungsmittel oder Lösungsmittelgemisch. Geeignete Lösungsmittel sind Alkohole, wie Methanol oder Aethanol, Aether, wie Tetrahydrofuran oder Dioxan, chlorierte Kohlenwasserstoffe, wie Methylenchlorid, und dergleichen. Für die basische Hydrolyse können Alkalimetallhydroxyde und -carbonate, wie Kalium- oder Natriumhydroxyd oder Kalium- oder Natriumcarbonat, organische Amine, wie Piperidin, und dergleichen verwendet werden. Inerte organische Lösungsmittel, wie sie oben für die saure Hydrolyse genannt sind, können als Lösungsvermittler

zugegeben werden. Die Reaktionstemperatur kann für die saure und basische Hydrolyse in einem Bereich von etwa 0° C bis Rückflusstemperatur variiert werden, wobei man vorzugsweise zwischen etwa 0° C und der Raumtemperatur arbeitet. Der t-Butoxycarbonylrest wird zweckmässig mit Trifluoressigsäure oder Ameisensäure in Gegenwart oder Abwesenheit eines inerten Lösungsmittels abgespalten. Die Fmoc-Schutzgruppe wird zweckmässig mit Piperidin bei etwa Raumtemperatur ab gespalten. Die Benzyloxycarbonylgruppe kann in bekannter Weise durch saure Hydrolyse wie weiter oben beschrieben oder hydrogenolytisch abgespalten werden.

Die Ausgangsstoffe der Formel II sind neu und ebenfalls Gegenstand der vorliegenden Erfindung. Diese Verbindungen können hergestellt werden, indem man eine Verbindung der Formel III mit gegebenenfalls N-methyliertem Histidin, Leucin, Norleucin, Norvalin, Thiazolylalanin, Thienylalanin, Asparaginsäureäthylester, Glutaminsäure-t-butylester, Glutaminsäurebenzylester oder t-Butoxyserin umsetzt. Diese Umsetzung erfolgt ebenfalls nach in der Peptid-Chemie bekannten Methoden, d.h. unter den Reaktionsbedingungen, wie sie weiter oben für die Umsetzung einer Verbindung der Formel II mit einem Dipeptid beschrieben sind.

Die Ausgangsstoffe der Formel III sind ebenfalls neu und Gegenstand der vorliegenden Erfindung. Sie können beispielsweise hergestellt werden, indem man in einer Verbindung der allgemeinen Formel

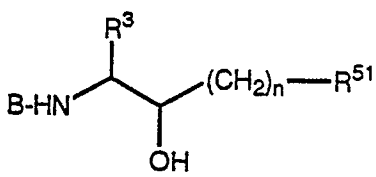

VI

oder

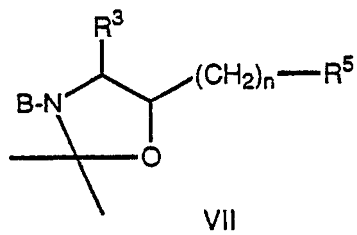

VII

worin B eine Aminoschutzgruppe, vorzugsweise t-Butoxycarbonyl oder Benzyloxycarbonyl, und $R^{51}$ Alkyl, Cycloalkyl, Alkenyl, Aryl oder Heteroaryl bedeuten und $R^3$ und $R^5$ die oben angegebene Bedeutung besitzen,
die Aminoschutzgruppe und gegebenenfalls gleichzeitig auch die O-Schutzgruppe abspaltet.

Die Abspaltung der N-Schutzgruppe und gegebenenfalls O-Schutzgruppe erfolgt ebenfalls nach an sich bekannten Methoden, beispielsweise in einem unter den Reaktionsbe dingungen inerten organischen Lösungsmittel oder Lösungsmittelgemisch bei einer Temperatur zwischen etwa 0° C und der Raumtemperatur mit einer Säure, wie Chlorwasserstoffsäure, Trifluoressigsäure, und dergleichen. Geeignete Lösungsmittel sind Aether, wie Tetrahydrofuran oder Dioxan, Alkohole, wie Methanol, oder chlorierte Kohlenwasserstoffe, wie Methylenchlorid, und dergleichen. Unter diesen Reaktionsbedingungen wird in einer Verbindung der Formel VII - wie bereits erwähnt - gleichzeitig der Oxazolidinring aufgespalten.

Die Ausgangsstoffe der Formel IV sind bekannt oder können in Analogie zur Herstellung der bekannten Verbindungen erhalten werden.

Die Verbindungen der Formeln VI und VII sind ebenfalls neu und Gegenstand vorliegender Erfindung. Die Verbindungen der Formel VI können beispielsweise durch Reduktion der entsprechenden Ketoverbindungen der allgemeinen Formel

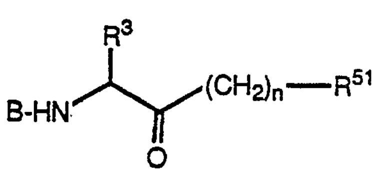

VIII

worin B, $R^3$ und $R^{51}$ die oben angegebene Bedeutung besitzen,
hergestellt werden.

Die Reduktion einer Ketoverbindung der Formel VIII erfolgt ebenfalls nach an sich bekannten Methoden, beispielsweise mit einem komplexen Metallhydrid, wie Natriumborhydrid und dergleichen, in einem unter den Reaktionsbedingungen inerten organischen Lösungsmittel oder Lösungsmittelgemisch bei einer Temperatur zwischen etwa 0 °C und etwa Raumtemperatur.

Die Verbindungen der Formel VII, worin $R^5$ Alkyl, Cycloalkyl, Alkenyl, Aryl oder Heteroaryl bedeutet, können beispielsweise durch Umsetzen einer Verbindung der Formel VI mit 2,2-Dimethoxypropan in Gegenwart von p-Toluolsulfonsäure hergestellt werden.

Die Verbindungen der Formel VII, worin $R^5$ Haloalkyl bedeutet, können beispielsweise hergestellt werden, indem man eine Verbindung der Formel VII, worin $R^5$ Alkenyl bedeutet, einer reduktiven bzw. oxydativen Ozonolyse unterwirft. Die bei der reduktiven Ozonolyse erhaltene Verbindung der allgemeinen Formel

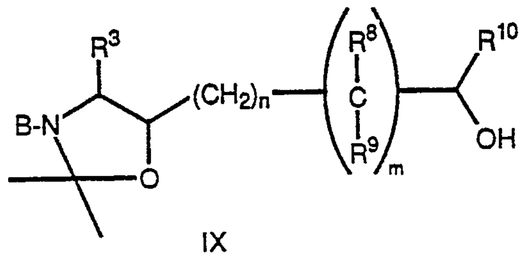

IX

worin $R^8$, $R^9$ und $R^{10}$ unabhängig voneinander je Wasserstoff oder Alkyl und m 0, 1, 2, 3, 4, 5, 6 oder 7 bedeuten, mit der Massgabe, dass die Gruppe

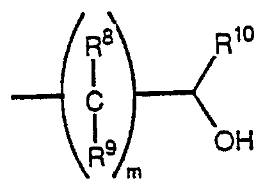

maximal 8 Kohlenstoffatome aufweist,
und B, $R^3$ und n die oben angegebene Bedeutung besitzen,
kann mit einem Halogenierungsmittel in eine Verbindung der Formel VII übergeführt werden, worin $R^5$ primäres oder sekundäres Haloalkyl bedeutet. Die bei der oxydativen Ozonolyse erhaltene Verbindung der allgemeinen Formel

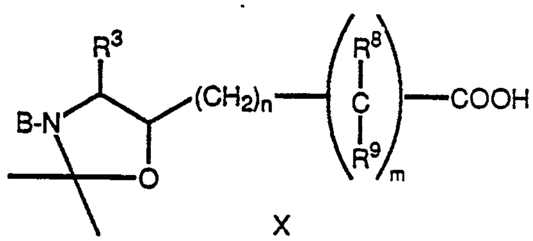

X

worin B, $R^3$, $R^8$, $R^9$, n und m die oben angegebene Bedeutung besitzen,
kann durch Umsetzen mit beispielsweise Diazomethan in den entsprechenden Methylester der allgemeinen Formel

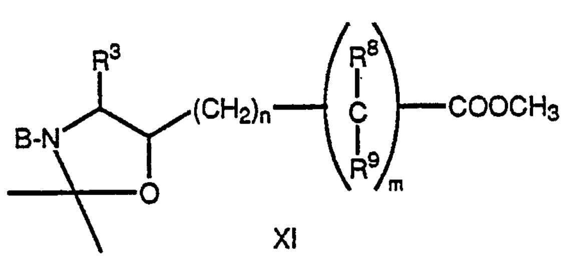

XI

worin B, $R^3$, $R^8$, $R^9$, n und m die oben angegebene Bedeutung besitzen,
übergeführt werden, welcher durch Umsetzen mit einer Verbindung der allgemeinen Formel
W-Mg-$R^{11}$ XII
worin $R^{11}$ Alkyl und W Chlor, Brom oder Jod, vorzugsweise Brom, bedeutet,
in einer Grignard-Reaktion in eine Verbindung der allgemeinen Formel

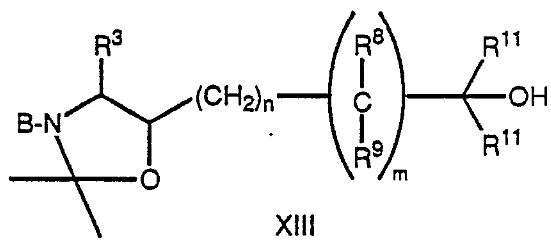

XIII

worin B, $R^3$, $R^8$, $R^9$, $R^{11}$, n und m die oben angegebene Bedeutung besitzen, mit der Massgabe, dass die Gruppe

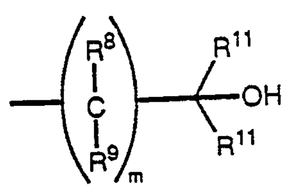

maximal 8 Kohlenstoffatome aufweist,
übergeführt werden kann. Diese Umsetzung erfolgt ebenfalls nach an sich bekannten Methoden, beispielsweise in einem unter den Reaktionsbedingungen inerten Lösungsmittel, wie einem Aether, bei einer Temperatur zwischen etwa 0 °C und 50 °C, vorzugsweise bei Raumtemperatur.

Umsetzen einer Verbindung der Formel XIII mit einem Halogenierungsmittel liefert eine Verbindung der Formel VII, worin $R^5$ tertiäres Haloalkyl bedeutet.

Die Ausgangsstoffe der Formel V sind ebenfalls neu und Gegenstand der vorliegenden Erfindung. Diejenigen, worin $R^{21}$ N-geschütztes Imidazol-2-yl, Imidazol-4-yl oder Pyrazol-3-yl bedeutet, können beispielsweise hergestellt werden, indem eine Verbindung der Formel III mit einer der Formel IV entsprechenden Verbindung, worin $R^2$ jedoch N-geschütztes Imidazol-2-yl, Imidazol-4-yl oder Pyrazol-3-yl bedeutet, oder einem aktivierten Derivat davon umsetzt. Die Umsetzung erfolgt nach an sich in der Peptid-Chemie bekannten Methoden, d.h. unter den gleichen Bedingungen wie oben für die Umsetzung einer Verbindung der Formel III mit einer Verbindung der Formel IV angegeben wurde.

Diejenigen Verbindungen der Formel V, worin $R^{41}$ N-geschütztes Amino bedeutet, können hergestellt werden, indem man beispielsweise eine Verbindung der allgemeinen Formel

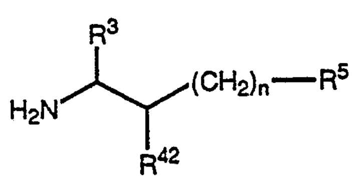

XIV

worin $R^{42}$ N-geschütztes Amino bedeutet und $R^3$, $R^5$ und n die oben angegebene Bedeutung besitzen,
mit einer Verbindung der Formel IV umsetzt. Auch diese Umsetzung erfolgt unter den oben für die Umsetzung einer Verbindung der Formel III mit einer Verbindung der Formel IV angegebenen Bedingungen.

Die Verbindungen der Formel XIV sind ebenfalls neu und Gegenstand vorliegender Erfindung. Diejenigen Verbindungen der Formel XIV, worin $R^5$ Alkyl, Cycloalkyl, Alkenyl, Aryl oder Heteroaryl bedeutet, können hergestellt werden, indem man beispielsweise eine Verbindung der Formel VIII mit Hydroxylamin zu

einer Verbindung der allgemeinen Formel

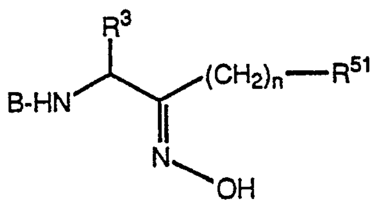

XV

worin B, $R^3$, $R^{51}$ und n die oben angegebene Bedeutung besitzen, umsetzt, diese zur entsprechenden Aminoverbindung der allgemeinen Formel

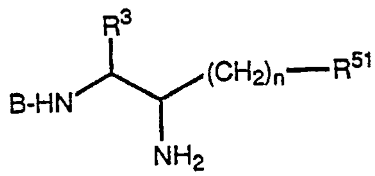

XVI

worin B, $R^3$, $R^{51}$ und n die oben angegebene Bedeutung besitzen, reduziert und die Aminogruppe unter Bildung einer Verbindung der allgemeinen Formel

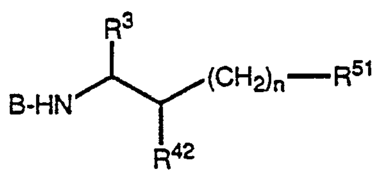

XVII

worin B, $R^3$, $R^{42}$, $R^{51}$ und n die oben angegebene Bedeutung besitzen, schützt. Durch Abspalten der Aminoschutzgruppe B unter den für die Verfahrensvariante d) angegebenen Bedingungen wird die entsprechende Verbindung der Formel XIV erhalten.

Die Verbindungen der Formel XIV, worin $R^5$ Haloalkyl bedeutet, können in analoger Weise wie oben für die Herstellung der Verbindungen der Formel VII, worin $R^5$ Haloalkyl bedeutet, beschrieben, aus denjenigen Verbindungen der Formel XVII hergestellt werden, worin $R^{51}$ Alkenyl bedeutet.

Die Verbindungen der Formeln VIII, IX, X, XI, XII, XIII, XV, XVI, XVII, XVIII, XIX, XX, XXI, XXII und XXIII sind bekannt oder können in Analogie zur Herstellung der bekannten Verbindungen erhalten werden.

Die verschiedenen Verfahren zur Herstellung der Verbindungen der Formeln III, V, VI und VII ausgehend von Verbindungen der Formel XVIII sind im nachfolgenden Schema I zusammengefasst. In diesem Schema bedeutet $R^{52}$ Haloalkyl. Bezüglich der genauen Reaktionsbedingungen sowie weiterer Herstellungsverfahren wird auf den Beispielteil verwiesen.

Schema I

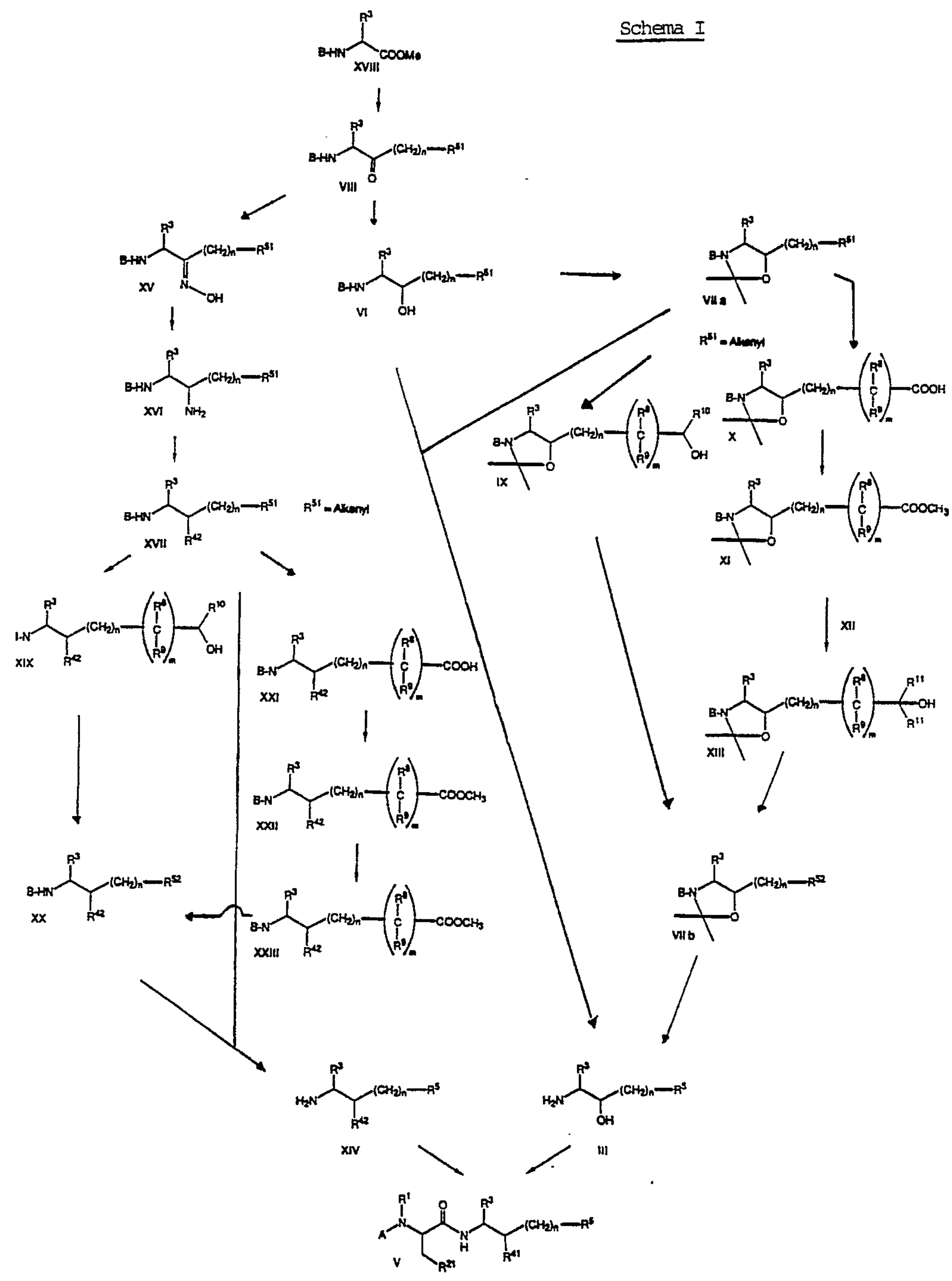

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven

Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

In vitro-Test mit reinem Human-Renin

Der Test wird in Eppendorf Röhrchen durchgeführt. Die Inkubationsmischung besteht aus (1) 100 µl Human-Renin in Puffer A (0,1M Natriumphosphatlösung, pH 7,4, enthaltend 0,1% Rinderserumalbumin, 0,1 % Natriumazid und 1 mM Aethylendiamintetraessigsäure), genügend für eine Renin-Aktivität von 2-3 ng Angiotensin I/ ml/Std.; (2) 145 µl Puffer A; (3) 30 µl von 10 µM humanem Tetradekapeptid-Reninsubstrat (hTD) in 10 mM Salzsäure; (4) 15 µl Dimethylsulfoxid mit bzw. ohne Hemmer und (5) 10 µl einer 0,03 molaren Lösung von Hydroxychinolinsulfat in Wasser.

Die Proben werden drei Stunden bei 37°C bzw. 4°C in Triplikaten inkubiert. 2 x 100 µl Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100 %; Angiotensin II 0,0013 %; hTD (Angiotensin I-Val-Ile-His-Ser-OH) 0,09 %. Die Produktion von Angiotensin I wird durch die Differenz zwischen dem Versuch bei 37°C und demjenigen bei 4°C bestimmt.

Folgende Kontrollen werden mitgeführt:

(a) Inkubation von hTD-Proben ohne Renin und ohne Hemmer bei 37°C und 4°C. Die Differenz zwischen diesen beiden Werten ergibt den Grundwert der Angiotensin I-Produktion.

(b) Inkubation von hTD-Proben mit Renin, jedoch ohne Hemmer bei 37°C und 4°C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus einer linearen Regressionskurve aus einem logit-log plot ermittelt.

Die in diesem Test erhaltenen Resultate sind in der folgenden Tabelle zusammengefasst:

Tabelle

| Verbindung | $IC_{50}$-Werte in µMol/lt. |
|---|---|
| A | 0,0002 |
| B | 0,001 |
| C | 0,0042 |
| D | 0,001 |
| E | 0,0026 |
| F | 0,001 |
| G | 0,005 |

A = t-Butyl (R)-2-[[(S)-α-[[(S)-1-[[(1S,2S,4R)-1-(cyclohexylmethyl)-2-hydroxy-4-äthyl-5-hexenyl]-carbamoyl]-2-imidazol-4-yläthyl]carbamoyl]phenäthyl]carbamoyl]-1-pyrrolidincarboxylat,

B = t-Butyl (R)-2-[[(S)-α-[[(S)-1-[[(1S,2S)-1-(cyclohexylmethyl)-2-hydroxy-4-äthylhexyl]carbamoyl]-2-imidazol-4-yläthyl]carbamoyl]phenäthyl]carbamoyl]-1-pyrrolidincarboxylat,

C = (S)-N-[(1S,2S,4R)-1-(Cyclohexylmethyl)-4-äthyl-2-hydroxy-5-hexenyl]-α-[(R)-α-(3,3-dimethyl-2-oxobutyl)hydrocinnamamido]imidazol-4-propionamid,

D = (S)-α-[(S)-α-[(t-Butylsulfonyl)methyl]hydrocinnamamido]-N-[(1S,2S,4R)-1-(cyclohexylmethyl)-4-äthyl-2-hydroxy-5-hexenyl]imidazol-5-propionamid,

E = t-Butyl [(S)-α-[[(S)-1-[[(1S,2R oder 2S)-3-cyclohexyl-1-(cyclohexylmethyl)-2-hydroxypropyl]-carbamoyl]-2-imidazol-4-yläthyl]carbamoyl]phenäthyl]carbamat,

F = (2R oder S,3S)-3-(Boc-D-Pro-Phe-His-NH)-1,4-dicyclohexyl-2-butanol und

G = (S)-N-[(1S,2S)-1-(Cyclohexylmethyl)-4-äthyl-4-fluor-2-hydroxyhexyl]-α-[(R)-α-(3,3-dimethyl-2-oxobutyl)hydrocinnamamido]imidazol-4-propionamid

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen verwendet:

Boc = t-Butoxycarbonyl
Fmoc = 9-Fluorenylmethoxycarbonyl
H-His-OH = L-Histidin
H-Phe-OH = L-Phenylalanin
H-D-Pro-OH = D-Prolin
H-Phe-His-OH = N-[(S)-2-Amino-3-phenylpropyl]-L-histidin
(Phe-His-NH) = L-Phenylalaninyl-L-histidinamido
$(Fmoc)_2$His-OH = N-α-N-im-Di-Fmoc-L-histidin

## Beispiel 1

Zu einer Lösung von 227 mg (0.5 mMol) (2)-α-Amino-N-[(1S,2R oder S)-1-(cyclohexylmethyl)-2-hydroxy-3-phenylpropyl]imidazol-4-propionamid-dihydrochlorid in 2 ml Dimethylformamid werden unter Rühren und Durchleiten von Argon bei 0° in der angegebenen Reihenfolge 151 mg N-Methylmorpholin, 146 mg Boc-Phe-OH und 208 mg HBTU gegeben. Danach lässt man das Reaktionsgemisch auf Raumtemperatur erwärmen und stellt durch tropfenweise Zugabe von N-Methylmorpholin den pH auf 8,5. Nach 2-stündigem Rühren bei Raumtemperatur wird das Reaktionsgemisch in gesättigte Natriumbicarbonatlösung gegossen und über Nacht bei Raumtemperatur gerührt. Danach wird das Gemisch mit Essigester extrahiert, und die organischen Extrakte mit Wasser gewaschen, über Magnesiumsulfat getrocknet und unter vermindertem Druck eingedampft. Das zurückbleibende braune Oel wird durch Chromatographie an Kieselgel mit einem 95:5-Gemisch von Essigester und Methanol als Eluierungsmittel gereinigt, wobei man 128 mg (41%)

t-Butyl [(S)-α-[[(S)-1-[[(1S,2R oder S)-1-(cyclohexylmethyl)-2-hydroxy-3-phenyl]carbamoyl]-2-imidazol-4-yläthyl]carbamoyl]phenäthyl]carbamat als weissen Festkörper erhält, der nach Umkristallisieren aus Aether bei 91°-93° schmilzt, MS: 632 (M + H)$^+$.

Das als Ausgangsstoff eingesetzte (2)-α-Amino-N-[(1S,2R oder S)-1-(cyclohexylmethyl)-2-hydroxy-3-phenylpropyl]imidazol-4-propionamid-dihydrochlorid wurde wie folgt hergestellt:

Eine Suspension von 1,45 g (0,06 Grammatom) Magnesiumspäne in 15 ml abs. Aether wird unter Rühren und Durchleiten von Argon mit einer Lösung von 10,26 g (0,06 mMol) Benzylbromid in 50 ml Aether tropfenweise so versetzt, dass das Reaktionsgemisch leicht unter Rückfluss siedet (35 Minuten). Nach beendeter Zugabe wird das Reaktionsgemisch 3 Stunden zum Rückfluss erhitzt bis alle Magnesiumspäne umgesetzt sind. Danach wird das Reaktionsgemisch auf -60° abge kühlt und innerhalb von 30 Minuten mit einer Lösung von 4,82g (19 mMol) 2-t-Butoxycarbonylamino-3(S)-cyclohexylpropylaldehyd, welcher nach der von J. Boger et al, in J. Med. Chem., 28, 1779 (1985) beschriebenen Methode hergestellt worden war, tropfenweise versetzt. Danach lässt man das Reaktionsgemisch auf Raumtemperatur erwärmen und giesst dieses in auf 0° gekühlte gesättigte Ammoniumchloridlösung. Die wässrige Phase wird mehrmals mit Aether extrahiert und die vereinigten Aetherextrakte mit gesättigter Ammoniumchloridlösung gewaschen, über Magnesiumsulfat getrocknet und unter vermindertem Druck eingedampft. Der ölige Rückstand wird durch Chromatographie an Kieselgel mit einem 4:1-Gemisch von Methylenchlorid und Essigester als Eluierungsmittel gereinigt. Kristallisation aus Hexan liefert 2,295 g (31.5%) t-Butyl [(1S,2R oder S)-1-(cyclohexylmethyl)-2-hydroxy-3-phenylpropyl]carbamat als weissen Festkörper, Schmelzpunkt 91°.

500 mg (1.4 mMol) t-Butyl [(1S,2R oder S)-1-(cyclohexylmethyl)-2-hydroxy-3-phenylpropyl]carbamat werden in 5 ml 4,6N Salzsäure in Methanol 2 Stunden bei Raumtemperatur stehen gelassen. Danach wird das Lösungsmittel unter vermindertem Druck abgedampft. Kristallisation des Rückstands aus Hexan liefert 369 mg (93%) (R oder S)-α-[(S)-1-Amino-2-cyclohexyläthyl]-2-phenyläthanol-hydrochlorid als weissen Festkörper, Schmelzpunkt 137°-142°.

In analoger Weise wie im ersten Absatz dieses Beispiels beschrieben, wurden durch Umsetzen von 1,42 g (5 mMol) (R oder S)-α-[(S)-1-Amino-2-cyclohexyläthyl]-2-phenyläthanol-hydrochlorid mit 1-(t-Butoxycarbonyl)-N-(t-butoxycarbonyl)-L-histidin 2,11 g (72%) t-Butyl [(S)-2-[1-(t-butoxycarbonyl)imidazol-4-yl]-1-[[(1S,2R oder S)-1-(cyclohexylmethyl)-2-hydroxy-3-phenylpropyl]carbamoyl]äthyl]carbamat als farblose Kristalle erhalten, Schmelzpunkt 98°-105° (aus Hexan), MS: 585 (M + H)$^+$.

1 g (1.7 mMol) t-Butyl [(S)-2-[1-(t-butoxycarbonyl)imidazol-4-yl]-1-[[(1S,2R oder S)-1-(cyclohexylmethyl)-2-hydroxy-3-phenylpropyl]carbamoyl]äthyl]carbamat wird in 5 ml 4,8N Salzsäure in Dioxan 6 Stunden unter Argon bei Raumtemperatur stehen gelassen. Eindampfen des Reaktionsgemisches zur Trockene liefert (2)-α-Amino-N-[(1S, 2R oder S)-1-(cyclohexylmethyl)-2-hydroxy-3-phenylpropyl]imidazol-4-propionamid-dihydrochlorid, welches ohne weitere Reinigung in die nächste Stufe eingesetzt wird.

## Beispiel 2

In analoger Weise wie in Beispiel 1 beschrieben, wurde durch Umsetzen von (S)-α-Amino-N-[(1S,2R oder S)-3-cyclohexyl-1-(cylcohexylmethyl)-2-hydroxypropyl]imidazol-4-propionamid-dihydrochlorid mit (R)-α-(Pivaloylmethyl)hydrozimtsäure (vgl. EPA 0.184.550) das (S)-N-[(1S,2R oder S)-3-Cyclohexyl-1-(cyclohexylmethyl)-2-hydroxypropyl]-α-[(R)-α-(3,3-dimethyl-2-oxobutyl)hydrocinnamamido]imidazol-4-propionamid in 40%iger Ausbeute als weisser Festkörper erhalten, Schmelzpunkt 164°-165° (aus Essigester/Isopropyläther), MS: 621 (M + H)$^+$.

Das als Ausgangsstoff eingesetzte (S)-α-Amino-N-[(1S,2R oder S)-3-cyclohexyl-1-(cylcohexylmethyl)-2-hydroxypropyl]imidazol-4-propionamid-dihydrochlorid wurde wie folgt hergestellt:

Eine Lösung von 4,7 g (13.5 mMol) t-Butyl [(1S,2R oder S)-1-(cyclohexylmethyl)-2-hydroxy-3-phenylpropyl]carbamat in 100 ml Methanol wird in Gegenwart von 5 g Rhodium auf Aluminiumoxyd (5%) bei Raumtemperatur und einem Druck von 350 kPa hydriert. Danach wird der Katalysator abfiltriert, und das Filtrat unter vermindertem Druck eingedampft, wobei man 4.7 g (98%) t-Butyl [(1S,2R oder S)-3-cyclohexyl-1-(cyclohexylmethyl)-2-hydroxypropyl]carbamat als weissen Schaum erhält, der ohne weitere Reinigung in die nächste Stufe eingesetzt wird, MS: 298 (M - Isobutyliden) 226 (M - Cyclohexyläthanol).

Eine Lösung von 4,0 g (11 mMol) t-Butyl [(1S,2R oder S)-3-cyclohexyl-1-(cyclohexylmethyl)-2-hydroxypropyl]carbamat in 10 ml Essigester und 20 ml 2,5N Salzsäure in Essigester wird unter Durchleiten von Argon 2 Stunden bei Raumtemperatur stehen gelassen, und danach wird das Lösungsmittel unter vermindertem Druck abgedampft. Umkristallisation des erhaltenen kristallinen Rückstands aus Aether/Hexan liefert 2,42 g (76%) (2R oder S)-3-Amino-1,4-dicyclohexyl-2-butanol-hydrochlorid als farblose Kristalle,

Schmelzpunkt 188°-190°.

In analoger Weise wie in Beispiel 1 beschrieben, wurden durch Umsetzen von 2 g (7 mMol) (2R oder S)-3-Amino-1,4-dicyclohexyl-2-butanol-hydrochlorid mit 1-(t-Butoxycarbonyl)-N-(t-butoxycarbonyl)-L-histidin 0,80 g (19,4%) t-Butyl 4-[(S)-2-(1-t-butoxyformamido)-2-[[(1S,2R oder S)-3-cyclohexyl-1-(cyclohexylmethyl)-2-hydroxypropyl]carbamoyl]äthyl]imidazol-1-carboxylat als farblose Flüssigkeit und 3,0 g (72,6%) des entsprechenden Diastereomerengemisches als farbloser Schaum erhalten. Das t-Butyl 4-[(S)-2-(1-t-butoxyformamido)-2-[[(1S,2R oder S)-3-cyclohexyl-1-(cyclohexylmethyl)-2-hydroxypropyl]carbamoyl]äthyl]imidazol-1-carboxylat wurde ohne weitere Reinigung in die nächste Stufe eingesetzt.

In analoger Weise wie in Beispiel 1 beschrieben, wurde durch Abspalten der Boc-Schutzgruppe das (S)-α-Amino-N-[(1S,2R oder S)-3-cyclohexyl-1-(cylcohexylmethyl)-2-hydroxypropyl]imidazol-4-propionamid-dihydrochlorid in 73%iger Ausbeute als weisser Festkörper erhalten, Schmelzpunkt 196°-196° (Zers., aus Essigester).

## Beispiel 3

In analoger Weise wie in Beispiel 1 beschrieben, wurde durch Umsetzen von (S)-α-Amino-N-[(1S,2R oder S)-3-cyclohexyl-1-(cylcohexylmethyl)-2-hydroxypropyl]imidazol-4-propionamid-dihydrochlorid mit Boc-Phe-OH das t-Butyl [(S)-α-[[(S)-1-[[(1S,2R oder S)-3-cyclohexyl-1-(cyclohexylmethyl)-2-hydroxypropyl]-carbamoyl]-2-imidazol-4-yläthyl]carbamoyl]phenäthyl]carbamat in 49%iger Ausbeute als weisser Festkörper erhalten, Schmelzpunkt 183°-184° (aus Hexan), MS: 638 $(M + H)^+$.

## Beispiel 4

In analoger Weise wie in Beispiel 1 beschrieben, wurde durch Abspalten der Boc-Schutzgruppe aus t-Butyl [(S)-α-[[(S)-1-[[(1S,2R oder S)-3-cyclohexyl-1-(cyclohexylmethyl)-2-hydroxypropyl]carbamoyl]-2-imidazol-4-yläthyl]carbamoyl]phenäthyl]carbamat das (2R oder 2S,3S)-1,4-Dicyclohexyl-3-(Phe-His-NH)-2-butanol in 86%iger Ausbeute als weisser Festkörper erhalten, Schmelzpunkt 193°-195° (Zers., aus Aether), MS: 538 $(M + H)^+$.

## Beispiel 5

In analoger Weise wie in Beispiel 1 beschrieben, wurde durch Umsetzen von (2R oder 2S,3S)-1,4-Dicyclohexyl-3-(Phe-His-NH)-2-butanol mit Boc-D-Pro-OH das (2R oder S,3S)-3-(Boc-D-Pro-Phe-His-NH)-1,4-dicyclohexyl-2-butanol in 40%iger Ausbeute als weisser Festkörper erhalten, Schmelzpunkt 131°-133° (aus Essigester/Hexan), MS: 735 $(M + H)^+$.

## Beispiel 6

In analoger Weise wie in Beispiel 1 beschrieben, wurde durch Umsetzen von (S)-α-Amino-N-[(1S,2R oder S)-1-(cyclohexylmethyl)-2-hydroxyphenäthyl]imidazol-4-propionamid mit (R)-α-(Pivaloylmethyl)-hydrozimtsäure das (S)-N-[(αS,βR oder S)-α-(Cyclohexylmethyl)-β-hydroxyphenäthyl]-α-[(R)-α-(3,3-dimethyl-2-oxobutyl)hydrocinnamamido]imidazol-4-propionamid in 22%iger Ausbeute als weisser Festkörper erhalten, Schmelzpunkt 95° (Zers., aus Essigester/Hexan), MS: 601 $(M + H)^+$.

Das als Ausgangsstoff eingesetzte (S)-α-Amino-N-[(1S,2R oder S)-1-(cyclohexylmethyl)-2-hydroxyphenäthyl]imidazol-4-propionamid wurde, in analoger Weise wie in Beispiel 1 beschrieben, wie folgt hergestellt:

Umsetzen von 2-t-Butoxycarbonylamino-3(S)-cyclohexylpropylaldehyd mit Phenylmagnesiumbromid in einer Grignard-Reaktion liefert das t-Butyl [(αS)-α-(cyclohexylmethyl)-β-hydroxyphenäthyl]carbamat in 34%iger Ausbeute als weissen Festkörper, Schmelzpunkt 108° (aus Hexan), MS: 260 (M -t-Butoxy), 226 (M - Benzylalkohol). Abspalten der Boc-Schutzgruppe liefert α-[(S)-1-Amino-2-cyclohexyläthyl]benzylalkohol-hydrochlorid in 87%iger Ausbeute als weissen Festkörper, Schmelzpunkt 172° (aus Hexan), MS: 234 (M + H)$^+$, welches durch Umsetzen mit 1-(t-Butoxycarbonyl)-N-(t-butoxycarbonyl)-L-histidin in 55%iger Ausbeute in das t-Butyl [(S)-2-[1-(t-butoxycarbonyl)imidazol-4-yl]-1-[[(αS,βR oder S)-α-(cyclohexylmethyl)-β-hydroxyphenäthyl]carbamoyl]äthyl]carbamat, Schmelzpunkt 97° (aus Hexan), MS: 571 (M + H)$^+$, übergeführt werden kann, aus welchem durch Abspalten der Boc-Schutzgruppe das (S)-α-Amino-N-[(1S,2R oder S)-1-(cyclohexylmethyl)-2-hydroxyphenäthyl]imidazol-4-propionamid als amorpher Festkörper in 74%iger Ausbeute erhalten wird, Schmelzpunkt 197°-199° (aus Hexan), MS: 371 (M + H)$^+$.

## Beispiel 7

In analoger Weise wie in Beispiel 1 beschrieben, wurde durch Umsetzen von (S)-α-Amino-N-[(2-cyclohexyl-1-(cyclohexylmethyl)-2-hydroxyäthyl]imidazol-4-propionamid-hydrochlorid (2:5) mit (R)-α-(Pivaloylmethyl)hydrozimtsäure das (S)-N-[(1S,2R oder S)-2-Cyclohexyl-1-(cyclohexylmethyl)-2-hydroxyäthyl]-α-[(R)-α-(3,3-dimethyl-2-oxobutyl)hydrocinnamamido]imidazol-4-propionamid in 20%iger Ausbeute als weisser Festkörper erhalten, Schmelzpunkt 97° (aus Hexan), MS: 607 (M + H)$^+$.

Das als Ausgangsstoff eingesetzte (S)-α-Amino-N-[(2-cyclohexyl-1-(cyclohexylmethyl)-2-hydroxyäthyl]-imidazol-4-propionamid-hydrochlorid (2:5) wurde wie folgt hergestellt:

Katalytische Hydrierung von t-Butyl [(αS)-α-(cyclohexylmethyl)-β-hydroxyphenäthyl]carbamat in analoger Weise wie in Beispiel 2 beschrieben, liefert das t-Butyl [(1S,2R oder S)-2-cyclohexyl-1-(cyclohexylmethyl)-2-hydroxyäthyl]carbamat in 93%iger Ausbeute als farblosen Schaum, MS: 266 (M -t-Butoxy), 226 (M - Cyclohexylmethanol), welches durch Abspalten der Boc-Schutzgruppe mit Salzsäure in Dioxan in analoger Weise wie in Beispiel 1 beschrieben, (1R oder S,2S)-2-Amino-1,3-dicyclohexyl-1-propanol-hydrochlorid in 90%iger Ausbeute als weissen Festkörper liefert, Schmelzpunkt 212° (aus Hexan). Umsetzen dieser Verbindung, in analoger Weise wie in Beispiel 1 beschrieben, mit 1-(t-Butoxycarbonyl)-N-(t-butoxycarbonyl)-L-histidin liefert das t-Butyl [(S)-2-[1-(t-butoxycarbonyl)imidazol-4-yl]-1-[[(1S,2R oder S)-2-cyclohexyl-1-(cyclohexylmethyl)äthyl]carbamoyl]äthyl]carbamat in 70%iger Ausbeute als farblosen Schaum, MS: 577 (M + H)$^+$, aus dem durch erneutes Abspalten der Boc-Schutzgruppe das (S)-α-Amino-N-[(2-cyclohexyl-1-(cyclohexylmethyl)-2-hydroxyäthyl]imidazol-4-propionamid-hydrochlorid (2:5) in 72%iger Ausbeute als weisser Festkörper hergestellt werden kann, Schmelzpunkt 175° (Zers., aus Hexan), MS: 358 (M - $H_2O$).

## Beispiel 8

In analoger Weise wie in Beispiel 1 beschrieben, wurde durch Umsetzen von (S)-α-Amino-N-[(2-cyclohexyl-1-(cyclohexylmethyl)-2-hydroxyäthyl]imidazol-4-propionamid-hydrochlorid (2:5) mit Boc-Phe-OH das t-Butyl [(S)-α-[[(S)-1-[[2-cyclohexyl-1-(cyclohexylmethyl)-2-hydroxyäthyl]carbamoyl]-2-imidazol-4-yläthyl]carbamoyl]phenäthyl]carbamat in 20%iger Ausbeute als weisser Festkörper erhalten, Schmelzpunkt 124°-127° (aus Essigester/Hexan), MS: 624 (M + H)$^+$.

Das als Ausgangsstoff eingesetzte (S)-α-Amino-N-[(2-cyclohexyl-1-(cyclohexylmethyl)-2-hydroxyäthyl]-imidazol-4-propionamid-hydrochlorid (2:5) wurde, in analoger Weise wie in Beispiel 1 beschrieben, wie folgt hergestellt:

Umsetzen von 2-t-Butoxycarbonylamino-3(S)-cyclohexylpropylaldehyd mit Cyclohexylmagnesiumbromid in einer Grignard-Reaktion liefert in 59%iger Ausbeute das t-Butyl [(2-cyclohexyl-1-(cyclohexylmethyl)-2-hydroxyäthyl]carbamat als Diastereomerengemisch in Form eines farblosen Oels, MS: 284 (M - Isobutyliden), 226 (M - Cyclohexylmethanol), welches nach Abspaltung der Boc-Schutzgruppe mit Salzsäure in Dioxan ein Diastereomerengemisch von 2-Amino-1,3-dicyclohexyl-1-propanol-hydrochlorid in 57%iger Ausbeute liefert, Schmelzpunkt 191°-198° (aus Hexan) MS: 240 (M + H)$^+$, 156 (M - Cyclohexan), 126 (M - Cyclohexylmethanol). Durch Umsetzen mit 1-(t-Butoxycarbonyl)-N-(t-butoxycarbonyl)-L-histidin erhält man in 48%iger Ausbeute das t-Butyl [(S)-2-[1-(t-butoxycarbonyl)imidazol-4-yl]-1-[[2-cyclohexyl-1-(cyclohexylmethyl)äthyl]carbamoyl]äthyl]carbamat als weissen Schaum, MS: 577 (M + H)$^+$. Abspalten der Boc-Schutzgruppe aus der eben genannten Verbindung liefert das (S)-α-Amino-N-[(2-cyclohexyl-1-(cyclohexylmethyl)-2-hydroxyäthyl]imidazol-4-propionamid-hydrochlorid (2:5) in 72%iger Ausbeute als weissen Festkörper, Schmelzpunkt 175° (Zers., aus Hexan), MS: 377 (M + H)$^+$.

Beispiel 9

740 mg (1 mMol) (S)-N-[(1S,2S)-1-(Cyclohexylmethyl)-4-äthyl-4-fluor-2-hydroxyhexyl]-α-[(R)-α-(3,3-dimethyl-2-oxobutyl)hyrocinnamamido]-1-(t-butoxycarbonyl)imidazol-4-propionamid werden in 10 ml Methanol gelöst, mit 10 mg Kaliumcarbonat versetzt und 1,5 Stunden bei Raumtemperatur gerührt. Danach wird das Reaktionsgemisch eingedampft, und der Rückstand an 50 g Kieselgel mit einem 1000:50:1-Gemisch von Chloroform, Aethanol und Ammoniak als Eluierungsmittel chromatographiert, wobei man 57 mg (9 %) (R)-N-[(1S,2S)-1-(Cyclohexylmethyl)-4-äthyl-4-fluor-2-hydroxyhexyl]-α-[(R)-α-(3,3-dimethyl-2-oxobutyl)-hydrocinnamamido]imidazol-4-propionamid als Oel und 317 mg (50 %) der epimeren Verbindung (S)-N-[-(1S,2S)-1-(Cyclohexylmethyl)-4-äthyl-4-fluor-2-hydroxyhexyl]-α-[(R)-α-(3,3-dimethyl-2-oxobutyl)-hydrocinnamamido]imidazol-4-propionamid als Schaum erhält, MS (jeweils): 627 $(M + H)^+$.

Das als Ausgangsstoff eingesetzte (S)-N-[(1S,2S)-1-(Cyclohexylmethyl)-4-äthyl-4-fluor-2-hydroxyhexyl]-α-[(R)-α-(3,3-dimethyl-2-oxobutyl)hyrocinnamamido]-1-(t-butoxycarbonyl)imidazol-4-propionamid wurde wie folgt hergestellt:

0,95 g (2.9 mMol) t-Butyl [(1S,2S)-1-(cyclohexylmethyl)-2-hydroxy-3-(methoxycarbonyl)propyl]carbamat, welches nach der in EPA 0.165.226 beschriebenen Methode hergestellt worden war, in 20 ml Dimethoxypropan und 50 mg (0.2 mMol) p-Toluolsulfonsäure werden 24 Stunden bei Raumtemperatur gerührt. Anschliessend wird das Reaktionsgemisch dreimal mit je 150 ml Aether extrahiert, und die Aetherextrakte mit Natriumbicarbonatlösung und gesättigter Kochsalzlösung gewaschen. Nach dem Trocknen und Eindampfen der Aetherextrakte wird der Rückstand mit einem 9:1-Gemisch von Toluol und Essigester als Eluierungsmittel an 80 g Kieselgel chromatographiert, wobei 900 mg (84 %) t-Butyl (4S,5S)-4-(cyclohexylmethyl)-5-[(methoxycarbonyl)methyl]-2,2-dimethyl-3-oxazolidincarboxylat erhalten werden, MS: 370 $(M + H)^+$.

850 mg (2,3 mMol) t-Butyl (4S,5S)-4-(cyclohexylmethyl)-5-[(methoxycarbonyl)methyl]-2,2-dimethyl-3-oxazolidincarboxylat in 15 ml Tetrahydrofuran werden tropfenweise bei Raumtemperatur zu einer aus 559 mg (23mGrammatom) Magnesiumspänen und 1,72 ml (23 mMol) Aethylbromid in 15 ml Tetrahydrofuran hergestellten Grignardlösung gegeben. Das Reaktionsgemisch wird anschliessend 1,5 Stunden bei Raumtemperatur weitergerührt, dann auf ein Gemisch von Eis und Ammoniumchloridlösung gegossen und dreimal mit je 150 ml Aether extrahiert. Die Aetherextrakte werden mit 70 ml Wasser gewaschen, vereinigt, getrocknet und eingedampft. Chromatographie des zurückbleibenden Oels (680 mg) werden mit einem 95:5-Gemisch von Toluol und Essigester, welches 1% Triäthylamin enthält, als Eluierungsmittel an 30 g Kieselgel chromatographiert, wobei man 600 mg (66 %) t-Butyl (4S,5S)-4-(cyclohexylmethyl)-5-(2-hydroxy-2-äthylbutyl)-2,2-dimethyl-5-oxazolidincarboxylat als Oel erhält, MS: 398 $(M + H)^+$.

Eine Lösung von 580 mg (1,45 mMol) t-Butyl (4S,5S)-4-(cyclohexylmethyl)-5-(2-hydroxy-2-äthylbutyl)-2,2-dimethyl-5-oxazolidincarboxylat in 2 ml Methylenchlorid wird bei -78° unter Argon zu einer Lösung von 0,53 ml (4,38 mMol) Diäthylaminoschwefeltrifluorid in 1 ml Methylenchlorid getropft, und das Reaktionsgemisch anschliessend 5 Stunden bei dieser Temperatur gerührt. Danach wird das Reaktionsgemisch zwischen Methylenchlorid und Wasser verteilt, und die organische Phase einmal mit Wasser gewaschen, über Magnesiumsulfat getrocknet und eingedampft. Der Rückstand (560 mg Oel) wird an 70 g Kieselgel mit einem 98:2-Gemisch von Toluol und Essigester als Eluierungsmittel chromatographiert, wobei 500 mg eines Oels erhalten werden, welches direkt in die nächste Stufe eingesetzt wird.

500 mg des obigen Oels werden 45 Minuten bei Raumtemperatur unter Argon in einer Lösung von 2,5 ml 4M Chlortrimethylsilan in Methylenchlorid und 7,5 ml 4M Phenol in Methylenchlorid verrührt. Danach wird das Reaktionsgemisch auf Eis gegossen und zweimal mit 150 ml Methylenchlorid extrahiert. Die organischen Extrakte werden mit 70 ml Wasser und 70 ml gesättigter Kochsalzlösung gewaschen, vereinigt, über Magnesiumsulfat getrocknet, filtriert und eingedampft. Chromatographie des zurückbleibenden Oels (1,66 g) an 70 g Kieselgel mit einem 20:1:0,1-Gemisch von Methylenchlorid, Methanol und Ammoniak als Eluierungsmittel liefert 177 mg (αS,βS)-β-Amino-α-(2-äthyl-2-fluorbutyl)cyclohexylpropanol als weissen Festkörper, MS: 260 $(M + H)^+$.

167 mg (0,64 mMol) (αS,βS)-ß-Amino-α-(2-äthyl-2-fluorbutyl)cyclohexylpropanol und 345 mg (0,71 mMol) 1-(t-Butoxycarbonyl)-N-[(R)-α-(3,3-dimethyl-2-oxobutyl)-hydrocinnamoyl]-L-histidin in 10 ml Dimethylformamid werden nacheinander mit 0,1 ml (0,71 mMol) Triäthylamin, 116 mg (0,71 mMol) HOBT und 289 mg (0,71 mMol) HBTU versetzt, und das Reaktionsgemisch 3 Stunden bei Raumtemperatur gerührt. Danach giesst man auf Eis und 70 ml 2N Natriumbicarbonatlösung und extrahiert dreimal mit je 150 ml Essigester. Die organischen Extrakte werden einmal mit Eis und 70 ml gesättigter Ammoniumchloridlösung und je einmal mit 70 ml 2N Natriumbicarbonatlösung und 70 ml gesättigter Kochsalzlösung gewaschen, über Magnesiumsulfat getrocknet, filtriert und eingedampft, wobei man 740 mg rohes (S)-N-[(1S,2S)-1-(Cyclohexylmethyl)-4-äthyl-4-fluor2-hydroxyhexyl]-α-[(R)-α-(3,3-dimethyl-2-oxobutyl)hydrocinnamamido]-1-(t-butoxycarbonyl)imidazol-4-propionamid erhält, welche ohne weitere Reinigung direkt in die nächste Stufe eingesetzt werden.

Das ebenfalls als Ausgangstoff eingesetzte 1-(t-Butoxycarbonyl)-N-[(R)-α-(3,3-dimethyl-2-oxobutyl)-hydrocinnamoyl]-L-histidin wurde wie folgt hergestellt:

Eine Suspension von 3,0 g (12 mMol) (R)-α-(Pivaloylmethyl)hydrozimtsäure und 2,66 g (11 mMol) L-Histidinmethylester-dihydrochlorid in 340 ml Dimethylformamid wird bei Raumtemperatur unter einer Stickstoffatmosphäre mit 3,45 g (34 mMol) Triäthylamin und 4,58 g (12 mMol) HBTU versetzt. Das Reaktionsgemisch wird 5 Stunden bei Raumtemperatur gerührt und anschliessend im Hochvakuum eingedampft. Der Rückstand wird in 500 ml Essigester gelöst und nacheinander mit 100 ml Wasser, dreimal je 100 ml gesättigter Natriumbicarbonatlösung und 100 ml gesättigter Natriumchloridlösung gewaschen. Die organische Phase wird über Natriumsulfat getrocknet, unter vermindertem Druck eingedampft, und das erhaltene gelbliche Rohprodukt an Kieselgel mit einem 95:5-Gemisch von Methylenchlorid und Methanol, welches 0,1% Ammoniak enthält, chromatographiert. Man erhält auf diese Weise 3,6 g N-[(R)-α-(3,3-Dimethyl-2-oxobutyl)hydrocinnamoyl]-L-histidinmethylester als farblosen Schaum, MS: 399 $(M)^+$.

Eine Lösung von 3,56 g (8,9 mMol) N-[(R)-α-(3,3-Dimethyl-2-oxobutyl)hydrocinnamoyl]-L-histidinmethylester und 9,36 ml 1N Natronlauge in 50 ml Methanol wird 15 Stunden bei Raumtemperatur gerührt und danach in der Kälte unter vermindertem Druck eingedampft. Man löst den Rückstand in 70 ml Dioxan und 30 ml Wasser, tropft bei Raumtemperatur eine Lösung von 2,95 (13,5 mMol) Di-t-butyldicarbonat zu und rührt danach 15 Stunden bei Raumtemperatur. Zur Aufarbeitung wird die Reaktionslösung unter vermindertem Druck auf ca. 1/3 ihres Volumens eingeengt und dann mit 200 ml Essigester verdünnt. Nach Zugabe von 50 ml Eiswasser wird das Reaktionsgemisch auf pH 2,5 gestellt, und die wässrige Phase mit festem Natriumchlorid gesättigt. Die wässrige Phase wird noch zweimal mit Essigester extrahiert, und die vereinigten Essigesterphasen über Natriumsulfat getrocknet und eingedampft. Das erhaltene Rohpodukt wird an Kieselgel mit einem 95:5-Gemisch von Methylenchlorid und Methanol, welches 0,1% Essigsäure enthält, chromatographiert, wobei man 3,5 g 1-(t-Butoxycarbonyl)-N-[(R)-α-(3,3-dimethyl-2-oxobutyl)-hydrocinnamoyl]-L-histidin als farbloses Pulver erhält, MS: 486 $(M+H)^+$

## Beispiel 10

200 mg (0,3 mMol) Benzyl [(S oder R)-1-[(S)-2-cyclohexyl-1-[(S)-α-[(R)-α-(3,3-dimethyl-2-oxobutyl)-hydrocinnamamido]imidazol-4-propionamido]äthyl]-2-äthylbutyl]carbamat werden in Gegenwart von 10 mg 10%igem Palladium auf Kohle in 30 ml Methanol 8 Stunden bei Raumtemperatur und Atmosphärendruck hydriert. Nach beendeter Wasserstoffaufnahme wird der Katalysator abfiltriert, und das Filtrat unter vermindertem Druck eingedampft, wobei 163 mg (100 %) (S)-N-[(1S,2S oder R)-2-Amino-1-(cyclohexylmethyl)-3-äthylpentyl]-α-[(R)-α-(3,3-dimethyl-2-oxobutyl)hydrocinnamamido]imidazol-4-propionamid in Form eines Festkörpers erhalten werden, MS: 594 $(M + H)^+$.

In analoger Weise wie oben beschrieben erhält man durch katalytische Hydrierung von 50 mg Benzyl [-(R oder S)-1-[(S)-2-cyclohexyl-1-[(S)-α-[(R)-α-(3,3-dimethyl-2-oxobutyl)hydrocinnamamido]imidazol-4-propionamido]äthyl]-2-äthylbutyl]carbamat in Gegenwart von 5 mg 10%igem Palladium auf Kohle in 15 ml Methanol 40 mg (98 %) (S)-N-[(1S,2R oder 2S)-2-Amino-1-(cyclohexylmethyl)-3-äthylpentyl]-α-[(R)-α-(3,3-dimethyl-2-oxobutyl)hydrocinnamamido]imidazol-4-propionamid als Schaum, MS: 594 $(M + H)^+$.

Die als Ausgangsstoffe eingesetzten Benzyl [(S oder R)-1-[(S)-2-cyclohexyl-1-[(S)-α-[(R)-α-(3,3-dimethyl-2-oxobutyl)hydrocinnamamido]imidazol-4-propionamido]äthyl]-2-äthylbutyl]carbamat und Benzyl [-(R oder S)-1-[(S)-2-cyclohexyl-1-[(S)-α-[(R)-α-(3,3-dimethyl-2-oxobutyl)hydrocinnamamido]imidazol-4-propionamido]äthyl]-2-äthylbutyl]carbamat wurden wie folgt hergestellt:

2,28 g (8 mMol) t-Butyl [(S)-1-(cyclohexylmethyl)-1-(methoxycarbonyl)methyl]carbamat [vgl. J. Boger et al., J. Med. Chem. 28, 1779 (1985)] in 30 ml Tetrahydrofuran werden bei Raumtemperatur tropfenweise zu einer aus 0,61 g (25 mGrammatom) Magnesiumspänen und 3,12 ml (25 mMol) 3-Brompentan in 35 ml Tetrahydrofuran hergestellten Grignardlösung gegeben, und das Reaktionsgemisch wird über Nacht bei Raumtemperatur weitergerührt. Anschliessend wird das Gemisch unter Kühlen in einem Eisbad tropfenweise mit ca. 40 ml gesättigter Ammoniumchloridlösung versetzt und dreimal mit je 300 ml Aether extrahiert. Die organischen Extrakte werden mit 100 ml Wasser und 100 ml gesättiger Kochsalzlösung gewaschen, vereinigt, getrocknet und eingedampft. Chromatographie des Rückstands (2,95 g) an 250 g Kieselgel mit einem 9:1-Gemisch von Hexan und Aether als Eluierungsmittel liefert 670 mg (26 %) t-Butyl [(S)-1-(cyclohexylmethyl)-3-äthyl-2-oxopentyl]carbamat als gelbes Oel, MS: 336 $(M + H)^+$.

Ein Gemisch von 550 mg (1,66 mMol) t-Butyl [(S)-1-(cyclohexylmethyl)-3-äthyl-2-oxopentyl]carbamat , 3 ml Pyridin, 1,15 g (1,66 mMol) Hydroxylaminhydrochlorid und 1,01 g 4-Dimethylaminopyridin wird unter Argon 5 Stunden auf 100° erhitzt. Danach wird das Reaktionsgemisch am Hochvakuum zur Trockene eingedampft, und der Rückstand dreimal mit je 130 ml Aether extrahiert. Die organischen Extrakte werden nacheinander mit 70 ml 2N Natriumbicarbonatlösung, 20%iger Kupfersulfatlösung und Wasser gewaschen, getrocknet und unter vermindertem Druck eingedampft. Der verbleibende Rückstand (540 mg) in 100 ml 3,5N methanolischen Ammoniak wird in Gegenwart von 1 g Raney-Nickel 2 Tage unter einer Wasserstoffatmosphäre gerührt. Danach wird der Katalysator abfiltriert, und das Filtrat unter vermindetem Druck eingedampft. Chromatographie des Rückstands an 50 g Kieselgel unter Verwendung eines 95:5-Gemisches von Chloroform und Aethanol als Eluierungsmittel liefert 260 mg (48 %) t-Butyl [(1S,2RS)-2-amino-1-(cyclohexylmethyl)-3-äthylpentyl]carbamat als Oel, MS: 327 $(M + H)^+$.

Ein Gemisch von 260 mg (0,8 mMol) t-Butyl [(1S,2RS)-2-amino-1-(cyclohexylmethyl)-3-äthylpentyl]-carbamat, 0,22 ml (1,60 mMol) Triäthylamin, 240 mg (0,96 mMol) N-(Benzyloxycarbonyloxy)succinimid und 15 ml Methylenchlorid wird 2 Stunden bei Raumtemperatur gerührt. Danach wird das Reaktionsgemisch unter vermindertem Druck eingedampft, und der Rückstand auf Eis gegossen und zweimal mit je 150 ml Aether extrahiert. Die organischen Extrakte werden mit 2N Natriumcarbonatlösung und Wasser gewaschen, vereinigt, getrocknet und eingedampft. Chromatographie des Rückstands an 50 g Kieselgel mit einem 97:3-Gemisch von Toluol und Essigester als Eluierungsmittel liefert die beiden epimeren Verbindungen 2-Benzyl 1-t-butyl [(1S,2S oder R)-1-(cyclohexylmethyl)-2-(1-äthylpropyl)äthylen]dicarbamat (240 mg) und 2-Benzyl 1-t-butyl [(1S,2R oder S)-1-(cyclohexylmethyl)-2-(1-äthylpropyl)äthylen]dicarbamat (80 mg), beide jeweils als Oel, MS (jeweils): 461 $(M + H)^+$.

In analoger Weise wie in Beispiel 9 beschrieben, wurden durch Umsetzen von 2-Benzyl 1-t-butyl [-(1S,2S oder R)-1-(cyclohexylmethyl)-2-(1-äthylpropyl)äthylen]dicarbamat und 2-Benzyl 1-t-butyl [(1S,2R oder S)-1-(cyclohexylmethyl)-2-(1-äthylpropyl)äthylen]dicarbamat mit 1-(t-Butoxycarbonyl)-N-[(R)-α-(3,3-dimethyl-2-oxobutyl)hydrocinnamoyl]-L-histidin und anschliessendem Abspalten der Boc-Schutzgruppe im Imidazolring mit Kaliumcarbonat in Methanol die beiden epimeren Verbindungen Benzyl [(S oder R)-1-[(S)-2-cyclohexyl-1-[(S)-α-[(R)-α-(3,3-dimethyl-2-oxobutyl)hydrocinnamamido]imidazol-4-propionamido]äthyl]-2-äthylbutyl]carbamat und Benzyl [(R oder S)-1-[(S)-2-cyclohexyl-1-[(S)-α-[(R)-α-(3,3-dimethyl-2-oxobutyl)-hydrocinnamamido]imidazol-4-propionamido]äthyl]-2-äthylbutyl]carbamat, beide jeweils als Schaum erhalten, MS (jeweils): 728 $(M + H)^+$.

## Beispiel 11

750 mg (1,19 mMol) (S)-α-Amino-N-[(1S,2RS)-1-(cyclohexylmethyl)-2-hydroxy-4,4-dimethylpentyl]-imidazol-4-propionamid und 337 mg (1,36 mMol) (R)-α-(Pivaloylmethyl)hydrozimtsäure werden, in analoger Weise wie nachstehend für die Herstellung des Ausgangsstoffes beschrieben, in 20 ml Dimethylformamid miteinander umgesetzt, und das Reaktionsgemisch aufgearbeitet. Chromatographie des Rückstands an 65 g Kieselgel mit einem 200:10:1-Gemisch von Methylenchlorid, Methanol und Ammoniak liefert 420 mg (59 %) (S)-N-[(1S,2RS)-1-(Cyclohexylmethyl)-2-hydroxy-4,4-dimethylpentyl]-α-[(R)-α-(3,3-dimethyl-2-oxobutyl)-hydrocinnamamido]imidazol-4-propionamid als amorphes Pulver, MS: 595 $(M + H)^+$.

Das als Ausgangstoff eingesetzte (S)-α-Amino-N-[(1S,2RS)-1-(cyclohexylmethyl)-2-hydroxy-4,4-dimethylpentyl]imidazol-4-propionamid wurde wie folgt hergestellt:

1,64 g (67 mGrammatom) Magnesiumspäne in Aether werden mit 8,85 ml (70 mMol) 1-Brom-2,2-dimethylpropan in 80 ml Aether tropfenweise so versetzt, dass das Reaktionsgemisch leicht unter Rückfluss siedet. Nach beendeter Zugabe wird das Reaktionsgemisch auf -60° abgekühlt und innerhalb von 30 Minuten mit einer Lösung von 5.1 g (20 mMol) 2-t-Butoxycarbonylamino-3(S)-cyclohexylpropylaldehyd in 60 ml Aether versetzt. Anschliessend wird das Reaktionsgemisch über Nacht bei Raumtemperatur gerührt, dann in einem Eisbad auf 5° abgekühlt und tropfenweise mit 25 ml gesättigter Ammoniumchloridlösung versetzt. Das Reaktionsgemisch wird danach dreimal mit 300 ml Aether extrahiert, und die Aetherextrakte mit gesättigter Ammoniumchloridlösung und Wasser gewaschen. Das nach dem Trocknen und Eindampfen verbleibende Oel (6,62 g) wird an 500 g Kieselgel mit zunächst einem 98:2- und anschliessend einem 95:5-Gemisch von Methylenchlorid und Essigester als Eluierungsmittel chromatographiert, wobei 4,11 g (63 %) t-Butyl [(1S,2RS)-1-(cyclohexylmethyl)-2-hydroxy-4,4-dimethylpentyl]carbamat als Oel erhalten werden, MS: 328 $(M + H)^+$.

4,11 g (12,5 mMol) t-Butyl [(1S,2RS)-1-(cyclohexylmethyl)-2-hydroxy-4,4-dimethylpentyl]carbamat werden 4 Stunden in 1,58M Salzsäure in Dioxan bei Raumtemperatur stehen gelassen. Danach wird das

Reaktionsgemisch unter vermindertem Druck zur Trockene eingedampft, wobei 1,24 g (38 %) (αRS,βS)-β-Amino-α-(2,2-dimethylpropyl)cyclohexylpropanol-hydrochlorid erhalten werden, welches ohne weitere Reinigung in die nächste Stufe eingesetzt wird.

Ein Gemisch von 1,24 g (4,7 mMol) (αRS,βS)-β-Amino-α-(2,2-dimethylpropyl)cyclohexylpropanol-hydrochlorid, 3,24 g (5,4 mMol) $(Fmoc)_2$His-OH, 1,78 ml (14,1 mMol) 4-Aethylmorpholin, 1,68 g (10,8 mMol) 87%iges HOBT, 1,244 g (6,49 mMol) EDC und 50 ml Dimethylformamid wird über Nacht bei Raumtemperatur stehen gelassen. Das Reaktionsgemisch wird danach unterhalb 40° am Hochvakuum eingeengt und dreimal mit 500 ml Essigester extrahiert. Die organischen Extrakte werden nacheinander mit 120 ml 2N Natriumbicarbonatlösung, 120 ml gesättigter Ammoniumchloridlösung, 120 2N Natriumbicarbonatlösung und 120 ml gesättigter Kochsalzlösung gewaschen. Der nach dem Trocknen und Eindampfen verbleibende Rückstand (4,7 g) wird in 100 ml Methylenchlorid gelöst und filtriert. Das Filtrat wird mit 2 ml Piperidin versetzt und 2 Stunden bei Raumtemperatur gerührt. Danach wird das Reaktionsgemisch eingedampft, und der Rückstand an Kieselgel chromatographiert, wobei 750 mg (44 %) (S)-α-Amino-N-[(1S,2RS)-1-(cyclohexylmethyl)-2-hydroxy-4,4-dimethylpentyl]imidazol-4-propionamid als dünnschichtchromatographisch einheitliches Produkt erhalten und direkt in die nächste Stufe eingesetzt werden.

## Beispiel 12

In analoger Weise wie in Beispiel 11 beschrieben, wurde durch Umsetzen von (S)-α-Amino-N-[(1S,2RS)-1-(cyclohexylmethyl)-2-hydroxy-3,3-dimethylbutyl]imidazol-4-propionamid mit (R)-α-(Pivaloylmethyl)hydrozimtsäure das N-[(1S,2RS)-1-(Cyclohexylmethyl)-2-hydroxy-3,3-dimethylbutyl]-α-[(R)-α-(3,3-dimethyl-2-oxobutyl)hydrocinnamamido]imidazol-4-propionamid als Schaum erhalten, MS: 581 $(M + H)^+$.

In analoger Weise wurde durch Umsetzen mit N-Isobutoxycarbonyl-L-phenylalanin anstelle der (R)-α-(Pivaloylmethyl)hydrozimtsäure das Isobutyl [(S)-α-[[(S)-1-[[(1S,2RS)-1-(cyclohexylmethyl)-2-hydroxy-3,3-dimethylbutyl]carbamoyl]-2-imidazol-4-yläthyl]carbamoyl]phenäthyl]carbamat als Schaum erhalten, MS: 598 $(M + H)^+$.

Das als Ausgangsstoff eingesetzte (S)-α-Amino-N-[(1S,2RS)-1-(cyclohexylmethyl)-2-hydroxy-3,3-dimethylbutyl]imidazol-4-propionamid wurde in Analogie zu Beispiel 11 wie folgt hergestellt:

Umsetzen von 2-t-Butoxycarbonylamino-3(S)-cyclohexylpropylaldehyd mit t-Butyllithium in Hexan anstelle der Umsetzung mit einer entsprechenden Grignardverbindung liefert das t-Butyl [(1S,2RS)-1-(cyclohexylmethyl)-2-hydroxy-3,3-dimethylbutyl]]carbamat als Oel, MS: 314 $(M + H)^+$, welches durch Abspalten der Boc-Schutzgruppe mit Salzsäure in Dioxan in das (αRS,βS)-β-Amino-α-(2,2-dimethyläthyl)-cyclohexylpropanol-hydrochlorid übergeführt werden kann, MS: 214 $(M + H)^+$. Umsetzen dieser Verbindung mit $(Fmoc)_2$His-OH und Abspalten der Fmoc-Schutzgruppen mit Piperidin liefert das (S)-α-Amino-N-[(1S,2RS)-1-(cyclohexylmethyl)-2-hydroxy-3,3-dimethylbutyl]imidazol-4-propionamid, welches ohne weitere Reinigung in die nächste Stufe eingesetzt wird.

## Beispiel 13

In analoger Weise wie in Beispiel 11 beschrieben, wurde durch Umsetzen von (S)-β-[(S)-1-Amino-2-cyclohexyläthyl]-2-benzimidazolhexanol mit 1-(t-Butoxycarbonyl)-N-[(R)-α-(3,3-dimethyl-2-oxobutyl)-hydrocinnamoyl]-L-histidin und Abspalten der Boc-Schutzgruppe mit Kaliumcarbonat in Methanol (S)-N-[(1S,2S)-7-(2-Benzimidazolyl)-1-(cyclohexylmethyl)-2-hydroxyheptyl]-α-[(R)-α-(3,3-dimethyl-2-oxobutyl) hydrocinnamamido]imidazol-4-propionamid in Form eines beige-farbenen amorphen Festkörpers erhalten, MS: 711 $(M + H)^+$.

Das als Ausgangsstoff eingesetzte (S)-β-[(S)-1-Amino-2-cyclohexyläthyl]-2-benzimidazolhexanol wurde wie folgt hergestellt:

Eine Lösung von 40,7 ml (0,3 Mol) 6-Brom-1-hexen in 270 ml Aether wird innerhalb von 75 Minuten unter Argon so zu 7,4 g (0.3 Grammatom) Magnesiumspänen in 60 ml Aether getropft, dass das Reaktionsgemisch leicht siedet. Nach beendeter Zugabe wird das Reaktionsgemisch noch 45 Minuten zum Rückfluss erhitzt, danach auf -60° abgekühlt und innerhalb von 1 Stunde mit 20 g (0,080 Mol) 2-t-Butoxycarbonylamino-3(S)-cyclohexylpropylaldehyd in 270 ml Aether bei -60° bis -70° versetzt. Nach beendeter Zugabe wird das Reaktionsgemisch 18 Stunden ohne Kühlung gerührt, danach wiederum auf 5°

abgekühlt und mit 400 ml gesättigter Ammoniumchloridlösung versetzt. Die beiden Phasen werden getrennt, und die organische Phase über Magnesiumsulfat getrocknet und eingedampft. Der Rückstand wird an 420 g Kieselgel mit Methylenchlorid und steigenden Anteilen an Essigester als Eluierungsmittel chromatographiert, wobei 19,3 g (71 %) t-Butyl [(1S,2S:R = 4:1)-1-(cyclohexylmethyl)-2-hydroxy-7-octenyl]-carbamat als Oel erhalten werden, MS: 284 (M - $C_4H_7$).

18,5 g (54,5 mMol) t-Butyl [(1S,2S:R = 4:1)-1-(cyclohexylmethyl)-2-hydroxy-7-octenyl]carbamat werden in 133 ml 2,2-Dimethoxypropan gelöst und mit 2,55 g (13 mMol) p-Toluolsulfonsäuremonohydrat versetzt. Danach wird das Reaktionsgemisch 4 Stunden bei Raumtemperatur gerührt, anschliessend auf ein Gemisch von Eiswasser und Natriumbicarbonatlösung gegossen und mit Essigester extrahiert. Die organischen Extrakte werden über Magnesiumsulfat getrocknet und eingedampft, wobei 20,2 g eines gelben Oels erhalten werden, welches an 200 g Kieselgel mit einem 9:1-Gemisch von Methylenchlorid und Hexan als Eluierungsmittel chroma tographiert wird. Dabei erhält man 19,0 g (92 %) t-Butyl (4S,5S:R = 4:1]-4-(cyclohexylmethyl)-5-(5-hexenyl)-2,2-dimethyl-3-oxazolidincarboxylat als gelbliches Oel, MS: 364 (M - $CH_3$).

Zu 19,0 g (50,1 mMol) t-Butyl (4S,5S:R = 4:1)-4-(cyclohexylmethyl)-5-(5-hexenyl)-2,2-dimethyl-3-oxazolidincarboxylat in 100 ml Hexan werden bei 0° innerhalb von 5 Minuten 2,5 ml (25 mMol) Borandimethylsulfitkomplex getropft. Danach wird das Reaktionsgemisch 10 Minuten bei 0° und anschliessend 3,5 Stunden bei Raumtemperatur gerührt. Danach kühlt man erneut ab und gibt zunächst bei 0°-5° innerhalb von 10 Minuten 16,8 ml Aethanol und anschliessend wiederum innerhalb von 10 Minuten 8,5 ml 2N Natronlauge tropfenweise zu. Nach 5-minütigem Rühren bei 5° werden 12,7 ml 30%ige Wasserstoffperoxidlösung innerhalb von 20 Minuten bei einer Temperatur von maximal 8° zugetropft. Danach wird das Reaktionsgemisch 2 Stunden zum Rückfluss erhitzt, abgekühlt, auf 500 ml Wasser gegossen und mit Methylenchlorid extrahiert. Die Methylenchloridextrakte werden über Magnesiumsulfat getrocknet und eingedampft, und der Rückstand an 240 g Kieselgel chromatographiert, wobei zuerst ein 98:2-Gemisch und dann ein 95:5-Gemisch von Methylenchlorid und Isopropanol als Eluierungsmittel verwendet wird. Auf diese Weise erhält man 12,8 g (64 %) t-Butyl (4S,5S)-4-(cyclohexylmethyl)-5-(6-hydroxyhexyl)-2,2-dimethyl-3-oxazolidincarboxylat als Isomerengemisch. Durch Kristallisation aus Aether/Hexan werden 7,2 g (36 %) t-Butyl (4S,5S)-4-(cyclohexylmethyl)-5-(6-hydroxyhexyl)-2,2-dimethyl-3-oxazolidincarboxylat als isomerenreine Verbindung erhalten, Schmelzpunkt 64° (aus Diäthyläther/Hexan).

4,0 g (10,1 mMol) t-Butyl (4S,5S)-4-(cyclohexylmethyl)-5-(6-hydroxyhexyl)-2,2-dimethyl-3-oxazolidincarboxylat werden in 60 ml Benzol gelöst und danach mit 7,95 g (50 mMol) pulverisiertem Kaliumpermanganat, 34 ml Wasser und 1,4 g Tricaprylmethylammoniumchlorid in 10 ml Benzol versetzt. Danach wird das Reaktionsgemisch auf 5° abgekühlt und mit 6,7 ml Eisessig versetzt. Dann wird das Reaktionsgemisch 3 Stunden bei Raumtemperatur intensiv gerührt, anschliessend auf 400 ml Eiswasser gegossen, durch Zugabe von Natriumpyrosulfit entfärbt und mit Methylenchlorid extrahiert. Die Methylenchloridextrakte werden über Magnesiumsulfat getrocknet und eingedampft. Der erhaltene Rückstand wird mit einem Gemisch von Methylenchlorid und Isopropanol als Eluierungsmittel an 60 g Kieselgel chromatographiert, wobei 3,4 g (82 %) (4S,5S)-3-(t-Butoxycarbonyl)-4-(cyclohexylmethyl)-2,2-dimethyl-5-oxazolidinhexansäure als farbloses, kristallisierendes Oel erhalten werden, welches direkt in die nächste Stufe eingesetzt wird.

1,2 g (2,9 mMol) (4S,5S)-3-(t-Butoxycarbonyl)-4-(cyclohexylmethyl)-2,2-dimethyl-5-oxazolidinhexansäure werden in 5 ml Tetrahydrofuran gelöst und mit 0,44 ml (3,1 mMol) Triäthylamin versetzt. Danach wird das Reaktionsgemisch auf -20° abgekühlt und innerhalb von 5 Minuten mit einer Lösung von 0,42 ml (0.31 mMol) Chlorameisensäureisobutylester in 1 ml Tetrahydrofuran versetzt. Nach 1-stündigem Rühren bei -20° wird eine Lösung von 0,63 g (5.8 mMol) 1,2-Phenylendiamin in 5 ml Tetrahydrofuran innerhalb von 5 Minuten bei -20° zugetropft. Danach wird das Reaktionsgemisch 30 Minuten bei -20° und dann 3,5 Stunden bei Raumtemperatur gerührt. Dann wird das Reaktionsgemisch auf Eiswasser gegossen und mit Methylenchlorid extrahiert, und die Methylenchloridextrakte über Magnesiumsulfat getrocknet und eingedampft. Der erhaltene Rückstand wird mit einem Gemisch von Methylenchlorid und Isopropanol als Eluierungsmittel an 20 g Kieselgel chromatographiert, wobei 1,4 g (96%) t-Butyl (4S,5S)-5-(5-[(o-aminophenyl)carbamoyl]pentyl]-4-(cyclohexylmethyl)-2,2-dimethyl-3-oxazolidincarboxylat als Oel erhalten werden, MS: 501 $(M + H)^+$.

1,4 g (2,79 mMol) t-Butyl (4S,5S)-5-(5-[(o-aminophenyl)carbamoyl]pentyl]-4-(cyclohexylmethyl)-2,2-dimethyl-3-oxozolidincarboxylat werden in 25 ml Methanol gelöst, auf 0° abgekühlt und mit 8,5 ml 3,88M Salzsäure in Methanol ver setzt. Anschliessend wird das Reaktionsgemisch 24 Stunden bei Raumtemperatur gerührt. Dann wird auf Eiswasser gegossen, mit 1N Natronlauge alkalisch gestellt und dreimal mit einem 4:1-Gemisch von Methylenchlorid und Isopropanol extrahiert. Die organischen Extrakte werden über Magnesiumsulfat getrocknet und eingedampft, und der Rückstand in einem Gemisch von 15 ml Methanol und 1,5 ml konz. wässrige Salzsäure 1 Stunde zum Rückfluss erhitzt. Das Reaktionsgemisch wird dann erneut wie oben beschrieben aufgearbeitet, und das erhaltene Rohprodukt in 20 ml wässriger 3N Salzsäure

2 Stunden zum Rückfluss erhitzt. Die nach einer dritten wie oben beschriebenen Aufarbeitung erhaltenen 760 mg Rohprodukt werden an 8 g Kieselgel mit Methylenchlorid und steigenden Anteilen an Isopropanol und wässrigem konzentriertem Ammoniak als Eluierungsmittel chromatographiert, wobei 346 mg (36%) (S)-$\beta$-[(S)-1-Amino-2-cyclohexyläthyl]-2-benzimidazolhexanol als Oel erhalten werden, MS: 344 $(M + H)^+$.

## Beispiel 14

0,35 g (1,33 mMol) Boc-Phe-OH, 0,28 g EDC und 0,21 g HOBT werden in 7,5 ml Methylenchlorid gelöst und bei -10° mit einer Lösung von 0,50 g (1,33 mMol) (S)-$\alpha$-Amino-N-[(1S,2S,4R)-1-(cyclohexylmethyl)-4-äthyl-2-hydroxy-5-hexenyl]imidazol-4-propionamid in 7,5 ml Methylenchlorid versetzt. Danach wird das Reaktionsgemisch 2 Stunden bei -10° und anschliessend 18 Stunden bei Raumtemperatur gerührt. Zur Aufarbeitung wird die Reaktionslösung auf 2N Natriumbicarbonatlösung gegossen und mit Methylenchlorid extrahiert. Die Methylenchloridextrakte werden über Kaliumcarbonat getrocknet und eingedampft, und der Rückstand an 10 g Kieselgel mit Methylenchlorid und steigenden Anteilen an Isopropanol als Eluierungsmittel chromatographiert. Auf diese Weise erhält man 0,73 g (88%) t-Butyl [(S)-$\alpha$-[[(S)-1-[[(1S,2S,4R)-1-cyclohexylmethyl)-2-hydroxy-4-äthyl-5-hexenyl]carbamoyl]-2-imidazol-4-yläthylcarbamoyl]-phenäthyl]carbamat als amorphen weissen Festkörper, MS: 624 $(M + H)^+$.

In analoger Weise wie oben beschrieben wird durch Umsetzen von (S)-$\alpha$-Amino-N-[(1S,2S,4S)-1-cyclohexylmethyl)-2-hydroxy-4-äthyl-5-hexenyl]imidazol-4-propionamid das t-Butyl [(S)-$\alpha$-[[(S)-1-[[(1S,2S,4S)-1-(cyclohexylmethyl)-2-hydroxy-4-äthyl-5-hexenyl]carbamoyl]-2-imidazol-4-yläthyl]carbamoyl]-2-imidazol-4-yläthyl]carbamoyl]phenäthyl]carbamat als amorpher weisser Festkörper erhalten, MS: 624 $(M + H)^+$.

Die als Ausgangsstoffe eingesetzten (S)-$\alpha$-Amino-N-[(1S,2S,4R)-1-(cyclohexylmethyl)-4-äthyl-2-hydroxy-5-hexenyl]imidazol-4-propionamid und (S)-$\alpha$-Amino-N-[(1S, 2S,4S)-1-cyclohexylmethyl)-2-hydroxy-4-äthyl-5-hexenyl]imidazol-4-propionamid wurden wie folgt hergestellt:

Eine Lösung von 44,9 g (0,276 mMol) 3-(Brommethyl)-1-penten, welches nach der von C. Jennings-White und R.G. Almquist in Tetrahedron Letters, 2533 (1982) beschriebenen Methode hergestellt worden war, in 225 ml Aether wird innerhalb von 90 Minuten zu 6,72 g (0,28 Grammatom) Magnesiumspänen in 75 ml Aether unter Argon so getropft, dass das Reaktionsgemisch leicht siedet. Nach beendeter Zugabe wird das Reaktionsgemisch 3,5 Stunden zum Rückfluss erhitzt, dann auf -60° abgekühlt und innerhalb von 50 Minuten mit einer Lösung von 22,6 g (0,075 Mol) 2-t-Butoxycarbonylamino-3(S)-cyclohexylpropylaldehyd in 225 ml Aether tropfenweise versetzt, wobei die Temperatur -60° bis -70° beträgt. Nach beendeter Zugabe wird das Kühlbad entfernt, und das Reaktionsgemisch 18 Stunden bei Raumtemperatur gerührt. Dann kühlt man auf 5° ab und gibt unter Rühren 100 ml einer gesättigten Ammoniumchloridlösung zu, wobei die Temperatur auf 20° ansteigt. Die beiden Phasen werden getrennt, die organische Phase über Magnesiumsulfat getrocknet und eingedampft, und der Rückstand an 1 kg Kieselgel mit Methylenchlorid, welches 0 bis 20% Essigester enthält, als Eluierungsmittel chromatographiert. Dabei erhält man 9,8 g (38,5%) ($\alpha$S,$\beta$S)-$\beta$-t-Butoxycarbonylamino-$\alpha$-[(R)-2-äthyl-3-butenyl]-cyclohexylpropanol, 2,7 g (11%) ($\alpha$S,$\beta$S)-$\beta$-t-Bu toxycarbonylamino-$\alpha$-[(S)-2-äthyl-3-butenyl]cyclohexylpropanol sowie 10,0 g (39%) eines Gemisches der beiden genannten Diastereomeren in Form von Oelen, welche ohne weitere Reinigung in die nächste Stufe eingesetzt werden.

3,5 g (10,3 mMol) ($\alpha$S,$\beta$S)-$\beta$-t-Butoxycarbonylamino-$\alpha$-[(R)-2-äthyl-3-butenyl]cyclohexylpropanol werden in einem Gemisch von 25 ml Methylenchlorid und 15 ml 90%iger Trifluoressigsäure 90 Minuten bei Raumtemperatur gerührt. Danach wird das Reaktionsgemisch auf Wasser gegossen, alkalisch gestellt und mit Methylenchlorid extrahiert. Die Methylenchloridextrakte werden über Magnesiumsulfat getrockent und eingedampft, und der Rückstand an 20 g Kieselgel mit einem 80:20:1-Gemisch von Methylenchlorid, Isopropanol und Ammoniak als Eluierungsmittel chromatographiert, wobei man 2,2 g (89%) (2S,3S,5R)-2-Amino-5-äthyl-1-cyclohexyl-6-hepten-3-ol als farbloses Oel erhält, MS: 142 $(M - C_7H_{13})$.

In analoger Weise erhält man durch Umsetzen von ($\alpha$S,$\beta$S)-$\beta$-t-Butoxycarbonylamino-$\alpha$-[(S)-2-äthyl-3-butenyl]cyclohexylpropanol mit Trifluoressigsäure das (2S,3S,5S)-2-Amino-5-äthyl-1-cyclohexyl-6-hepten-3-ol als gelblichen Festkörper, MS: 240 $(M+H)^+$.

2,2 g (9,19 mMol) (2S,3S,5R)-2-Amino-5-äthyl-1-cyclohexyl-6-hepten-3-ol in 20 ml Methylenchlorid werden bei -10° zu einer Lösung von 5,7 g (9,5 mMol) $(Fmoc)_2$His-OH, 2,2 g EDC und 1,75 g HOBT in einem Gemisch von 40 ml Dimethylformamid und 80 ml Methylenchlorid getropft. Nach beendeter Zugabe wird das Reaktionsgemisch 2 Stunden bei -10° und 16 Stunden bei Raumtemperatur gerührt, danach auf 2N Natriumbicarbonatlösung gegossen und mit Methylenchlorid extrahiert. Die Methylenchloridextrakte

werden über Kaliumcarbonat getrocknet und eingedampft. Der erhaltene Rückstand (7,1 g) wird in 100 ml Acetonitril suspendiert, mit 100 ml Diäthylamin versetzt und 17 Stunden bei Raumtemperatur gerührt. Danach wird das Reaktionsgemisch filtriert, das Filtrat eingedampft, und der Rückstand an 70 g Kieselgel mit Methylenchlorid und steigenden Anteilen an Isopropanol und gesättigtem wässrigem Ammoniak als Eluierungsmittel chromatographiert, wobei man 1,67 g (48%) (S)-α-Amino-N-[(1S,2S,4R)-1-(cyclohexylmethyl)-4-äthyl-2-hydroxy-5-hexenyl]imidazol-4-propionamid als Oel erhält, MS: 263 (M - $C_7H_{13}O$).

In analoger Weise wie oben beschrieben, wurde durch Umsetzen von (2S,3S,5S)-2-Amino-5-äthyl-1-cyclohexyl-6-hepten-3-ol mit $(Fmoc)_2$His-OH und Abspalten der Fmoc-Schutzgruppen mit Diäthylamin das (S)-α-Amino-N-[(1S, 2S,4S)-1-cyclohexylmethyl)-2-hydroxy-4-äthyl-5-hexenyl]imidazol-4-propionamid als gelblicher Festkörper erhalten, MS: 377 $(M+H)^+$.

## Beispiel 15

0,30 g (0,48 mMol) t-Butyl [(S)-α-[[(S)-1-[[(1S,2S,4R)-1-(cyclohexylmethyl)-2-hydroxy-4-äthyl-5-hexenyl]-carbamoyl]-2-imidazol-4-yläthylcarbamoyl]phenäthyl]carbamat in 3 ml Methanol wird in Gegenwart von 60 mg Palladium auf Kohle (5%) 7,5 Stunden bei Raumtemperatur und Atmosphärendruck hydriert. Nach beendeter Wasserstoffaufnahme wird der Katalysator abfiltriert, und das Filtrat eingedampft, wobei 0,20 g (66,5%) t-Butyl [(S)-α-[[(S)-1-[[(1S,2S)-1-(cyclohexylmethyl)-2-hydroxy-4-äthylhexyl]carbamoyl]-2-imidazol-4-yläthyl]carbamoyl]phenäthyl]carbamat als weisser amorpher Festkörper erhalten wird, MS: 626 $(M + H)^+$.

## Beispiel 16

0,55 g (1,05 mMol) (S)-N-[(1S,2S,4R)-1-(Cyclohexylmethyl)-2-hydroxy-4-äthyl-5-hexenyl]-α-(3-phenyl-L-alanyl)imidazol-4-propionamid in 5 ml Methylenchlorid wird bei -10° zu einer Lösung von 0,23 g (1,05 mMol) Boc-D-Pro-OH, 0,22 g EDC und 0,17 g HOBT in 5 ml Methylenchlorid getropft. Das Reaktionsgemisch wird nach beendeter Zugabe bei -10° 2 Stunden und bei Raumtemperatur 18 Stunden gerührt, danach auf 2N Natriumbicarbonatlösung gegossen und mit Methylenchlorid extrahiert. Die Methylenchloridextrakte werden über Kaliumcarbonat getrocknet und eingedampft, und der Rückstand an 10 g Kieselgel mit Methylenchlorid und steigenden Anteilen an Isopropanol als Eluierungsmittel chromatographiert, wobei 0,59 g (78%) t-Butyl (R)-2-[[(S)-α-[[(S)-1-[[(1S,2S,4R)-1-(cyclohexylmethyl)-2-hydroxy-4-äthyl-5-hexenyl]-carbamoyl]-2-imidazol-4-yläthyl]carbamoyl]phenäthyl]carbamoyl]-1-pyrrolidincarboxylat als amorpher Festkörper erhalten wird, MS: 721 $(M + H)^+$.

Das als Ausgangsstoff eingesetzte (S)-N-[(1S,2S,4R)-1-(Cyclohexylmethyl)-2-hydroxy-4-äthyl-5-hexenyl]-α-(3-phenyl-L-alanyl)imidazol-4-propionamid wurde wie folgt hergestellt:

0,60 g (1,59 mMol) (S)-α-Amino-N-[(1S,2S,4R)-1-(cyclohexylmethyl)-4-äthyl-2-hydroxy-5-hexenyl]-imidazol-4-propionamid in 7,5 ml Methylenchlorid wird bei -10° zu einer Lösung von 0,62 g (1,59 mMol) Fmoc-Phe-OH, 0,33 g EDC und 0,24 g HOBT in 7,5 ml Methylenchlorid getropft. Nach beendeter Zugabe wird das Reaktionsgemisch 2 Stunden bei -10° und anschliessend 17 Stunden bei Raumtemperatur gerührt, danach auf 2N Natriumbicarbonatlösung gegossen und mit Methylenchlorid extrahiet. Die Methylenchloridextrakte werden über Kaliumcarbonat getrocknet und eingedampft, und der Rückstand an 15 g Kieselgel mit Methylenchlorid und steigenden Anteilen an Isopropanol als Eluierungsmittel chromatographiert. Das so erhaltene kristalline Produkt (0,90 g) wird in 12,5 ml Acetonitril suspendiert und anschliessend mit 12,5 ml Diäthylamin versetzt. Danach wird das Reaktionsgemisch 75 Minuten bei Raumtemperatur gerührt und dann eingedampft, und der Rückstand an 10 g Kieselgel mit Methylenchlorid und steigenden Anteilen an Isopropanol und gesättigter Ammoniaklösung als Eluierungsmittel chromatographiert, wobei 0,62 g (74,5%) (S)-N-[(1S,2S,4R)- 1-(Cyclohexylmethyl)-2-hydroxy-4-äthyl-5-hexenyl]-α-(3-phenyl-L-alanyl)-imidazol-4-propionamid als amorpher, weisser Festkörper erhalten wird, MS: 524 $(M + H)^+$.

## Beispiel 17

In analoger Weise wie in Beispiel 15 beschrieben, wurde durch katalytische Hydrierung von t-Butyl (R)-2-[[(S)-α-[[(S)-1-[[(1S,2S,4R)-1-(cyclohexylmethyl)-2-hydroxy-4-äthyl-5-hexenyl]carbamoyl]-2-imidazol-4-yläthyl]carbamoyl]phenäthyl]carbamoyl]-1-pyrrolidincarboxylat das t-Butyl (R)-2-[[(S)-α-[[(S)-1-[[(1S,2S)-1-(cyclohexylmethyl)-2-hydroxy-4-äthylhexyl]carbamoyl]-2-imidazol-4-yläthyl]carbamoyl]phenäthyl]-carbamoyl]-1-pyrrolidincarboxylat als amorpher, weisser Festkörper erhalten, MS: 723 $(M + H)^+$.

## Beispiel 18

In analoger Weise wie in Beispiel 14 beschrieben, wurden die folgenden Verbindungen hergestellt:

- Aus (S)-α-Amino-N-[(1S,2S,4R)-1-(cyclohexylmethyl)-4-äthyl-2-hydroxy-5-hexenyl]imidazol-4-propionamid und (R)-α-(Pivaloylmethyl)hydrozimtsäure das (S)-N-[(1S,2S,4R)-1-(Cyclohexylmethyl)-4-äthyl-2-hydroxy-5-hexenyl]-α-[(R)-α-(3,3-dimethyl-2-oxobutyl)hydrocinnamamido]imidazol-4-propionamid als weisser Schaum, MS: 607 $(M + H)^+$ und
- aus (S)-α-Amino-N-[(1S,2S,4R)-1-(cyclohexylmethyl)-4-äthyl-2-hydroxy-5-hexenyl]imidazol-4-propionamid und α-[(t-Butylsulfonyl)methyl]hydrozimtsäure das (S)-α-[(S)-α-[(t-Butylsulfonyl)methyl]hydrocinnamamido]-N-[(1S,2S,4R)-1-(cyclohexylmethyl)-4-äthyl-2-hydroxy-5-hexenyl]imidazol-5-propionamid als Festkörper, MS: 657 $(M + H)^+$.

## Beispiel 19

In analoger Weise wie in Beispiel 15 beschrieben, wurde durch katalytische Hydrierung von (S)-α-[(S)-α-[(t-Butylsulfonyl)methyl]hydrocinnamamido]-N-[(1S,2S,4R)-1-(cyclohexylmethyl)-4-äthyl-2-hydroxy-5-hexenyl]imidazol-5-propionamid das (S)-α-[(S)-α-[(t-Butylsulfonyl)methyl]hydrocinnamamido]-N-[(1S,2S,4R)-1-(cyclohexylmethyl)-4-äthyl-2-hydroxyhexyl]imidazol-5-propionamid als amorpher, weisser Festkörper erhalten, MS: 645 $(M + H)^+$.

## Beispiel A

Eine sterilfiltrierte wässrige Lösung von t-Butyl (R)-2-[[(S)-α-[[(S)-1-[[(1S,2S,4R)-1-(cyclohexylmethyl)-2-hydroxy-4-äthyl-5-hexenyl]carbamoyl]-2-imidazol-4-yläthyl]carbamoyl]phenäthyl]carbamoyl]-1-pyrrolidincarboxylat wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| t-Butyl (R)-2-[[(S)-α-[[(S)-1-[[(1S,2S,4R)-1-(cyclohexylmethyl)-2-hydroxy-4-äthyl-5-hexenyl]carbamoyl]-2-imidazol-4-yläthyl]carbamoyl]phenäthyl]carbamoyl]-1-pyrrolidincarboxylat | 3,0 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel B

Man löst 5 mg t-Butyl (R)-2-[[(S)-α-[[(S)-1-[[(1S,2S,4R)-1-(cyclohexylmethyl)-2-hydroxy-4-äthyl-5-hexenyl]carbamoyl]-2-imidazol-4-yläthyl]carbamoyl]phenäthyl]carbamoyl]-1-pyrrolidincarboxylat in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel C

In einer Mischung von 3,5 ml Myglyol 812 und 0,08 g Benzylalkohol werden 500 mg fein gemahlenes (5,0 µm) t-Butyl (R)-2-[[(S)-α-[[(S)-1-[[(1S,2S,4R)-1-(cyclohexylmethyl)-2-hydroxy-4-äthyl-5-hexenyl]-carbamoyl]-2-imidazol-4-yläthyl]carbamoyl]phenäthyl]carbamoyl]-1-pyrrolidincarboxylat suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

Beispiel D

Wenn man nach den in den Beispielen A-C beschriebenen Verfahren arbeitet, können aus den folgenden, ebenfalls bevorzugten Verbindungen entsprechende galenische Präparate hergestellt werden:
t-Butyl (R)-2-[[(S)-α-[[(S)-1-[[(1S,2S)-1-(cyclohexylmethyl)-2-hydroxy-4-äthylhexyl]carbamoyl]-2-imidazol-4-yläthyl]carbamoyl]phenäthyl]carbamoyl]-1-pyrrolidincarboxylat
(S)-N-[(1S,2S,4R)-1-(Cyclohexylmethyl)-4-äthyl-2-hydroxy-5-hexenyl]-α-[(R)-α-(3,3-dimethyl-2-oxobutyl)-hydrocinnamamido]imidazol-4-propionamid,
(S)-α-[(S)-α-[(t-Butylsulfonyl)methyl]hydrocinnamamido]-N-[(1S,2S,4R)-1-(cyclohexylmethyl)-4-äthyl-2-hydroxy-5-hexenyl]imidazol-5-propionamid;
t-Butyl [(S)-α-[[(S)-1-[[(1S,2R oder 2S)-3-cyclohexyl-1-(cyclohexylmethyl)-2-hydroxypropyl]carbamoyl]-2-imidazol-4-yläthyl]carbamoyl]phenäthyl]carbamat;
(2R oder S,3S)-3-(Boc-D-Pro-Phe-His-NH)-1,4-dicyclohexyl-2-butanol und
(S)-N-[(1S,2S)-1-(Cylohexylmethyl)-4-äthyl-4-fluor-2-hydroxyhexyl]-α-[(R)-α-(3,3-dimethyl-2-oxobutyl)-hydrocinnamamido]imidazol-4-propionamid.

## Ansprüche

1. Aminosäurederivate der allgemeinen Formel

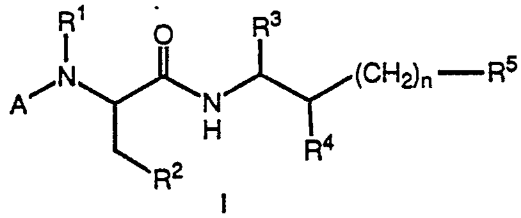

I

worin $R^1$ Wasserstoff oder Methyl, $R^2$ Aethyl, Propyl, Isopropyl, Imidazol-2-yl, Imidazol-4-yl, Pyrazol-3 yl,

Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Hydroxy oder Amino, $R^5$ Alkyl, Haloalkyl, Cycloalkyl, Alkenyl, Aryl oder Heteroaryl, n 0, 1, 2, 3, 4, 5 oder 6 und A eine der Gruppen

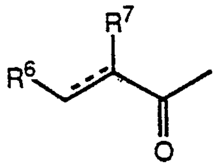 (a) und -Y-Z (b)

bedeuten, worin die gestrichelte Linie eine zusätzliche Bindung bedeuten kann, $R^6$ Phenyl, substituiertes Phenyl, Benzyl oder Naphthyl und $R^7$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxy phosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin und Z Wasserstoff oder Acyl bedeuten, mit den Massgaben, dass

(i) falls n 2, 3, 4, 5 oder 6 bedeutet, $R^5$ nicht Alkyl, Cycloalkyl oder Aryl bedeutet,

(ii) falls n 1 und $R^5$ Alkyl bedeuten, das an die $CH_2$-Gruppe gebundene Kohlenstoffatom von Alkyl verzweigt ist,

(iii) falls n 0 und $R^5$ Alkyl bedeuten, das an das den Substituenten $R^4$ tragende Kohlenstoffatom gebundene Kohlenstoffatom von $R^5$ in α-Stellung keine Methylengruppe aufweist,

(iv) falls $R^2$ Imidazol-4-yl, $R^4$ Hydroxy und Y Phenylalanin bedeuten, $R^5$ nicht Alkyl bedeutet und

(v) falls $R^6$ Phenyl, Benzyl oder α-Naphthyl bedeutet, $R^7$ nicht Alkoxycarbonylamino oder Arylalkoxycarbonylamino bedeutet,

in Form von optisch reinen Diastereomeren, Diastereomerengemischen, diastereomeren Racematen oder Gemischen von diastereomeren Racematen sowie pharmazeutisch verwendbare Salze dieser Verbindungen.

2. Verbindungen gemäss Anspruch 1, worin $R^1$ Wasserstoff bedeutet.

3. Verbindungen gemäss Anspruch 1 oder 2, worin $R^2$ Imidazol-2-yl, Imidazol-4-yl oder Thiazol-4-yl, insbesondere Imidazol-4-yl, bedeutet.

4. Verbindungen gemäss einem der Ansprüche 1-3, worin $R^3$ Cyclohexylmethyl bedeutet.

5. Verbindungen gemäss einem der Ansprüche 1-4, worin $R^4$ Hydroxy bedeutet.

6. Verbindungen gemäss einem der Ansprüche 1-5, worin $R^5$ Alkyl, Haloalkyl, Cycloalkyl oder Alkenyl, insbesondere 3-Pentyl, Fluoralkyl, Cyclohexyl oder 3-Pentenyl, bedeutet.

7. Verbindungen gemäss einem der Ansprüch 1-6, worin n 0 oder 1, insbesondere 1, ist.

8. Verbindungen gemäss einem der Ansprüche 1-7, worin A die Gruppe (a) bedeutet.

9. Verbindungen gemäss Anspruch 8, worin $R^6$ Phenyl oder substituiertes Phenyl, insbesondere Phenyl, bedeutet.

10. Verbindungen gemäss Anspruch 8 oder 9, worin $R^7$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, insbesondere Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeutet.

11. Verbindungen gemäss einem der Ansprüche 1-7, worin A die Gruppe (b) und Y den N-terminal mit Z verbundenen, bivalenten Rest von Phenylalanin bedeutet.

12. Verbindungen gemäss Anspruch 11, worin Z die Gruppe $R^a$-O-CO- oder den Rest einer durch diese Gruppe acylierten α-Aminosäure, insbesondere Prolin, bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, insbesondere einen gesättigten aliphatischen Kohlenwasserstoffrest mit bis zu 6 Kohlenstoffatomen, bedeutet.

13. Verbindungen gemäss Anspruch 1, worin $R^1$ Wasserstoff, $R^2$ Imidazol-4-yl, $R^3$ Cyclohexylmethyl, $R^4$ Hydroxyl, $R^5$ 3-Pentyl, Fluoralkyl, Cyclohexyl oder 3-Pentenyl, n 1, $R^6$ Phenyl, $R^7$ $C_1$-$C_4$-Alkylc-

arbonylmethyl oder $C_1$-$C_4$-Alkylsulfonylmethyl, Y den N-terminal mit Z verbundenen, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen bedeutet.

14. t-Butyl (R)-2-[[(S)-α-[[(S)-1-[[(1S,2S,4R)-1-(cyclohexylmethyl)-2-hydroxy-4-äthyl-5-hexenyl]-carbamoyl]-2-imidazol-4-yläthyl]carbamoyl]phenäthyl]carbamoyl]-1-pyrrolidincarboxylat, t-Butyl (R)-2-[[(S)-α-[[(S)-1-[[(1S,2S)-1-(cyclohexylmethyl)-2-hydroxy-4-äthylhexyl]carbamoyl]-2-imidazol-4-yläthyl]carbamoyl]-phenäthyl]carbamoyl]-1-pyrrolidincarboxylat, (S)-N-[(1S,2S,4R)-1-(Cyclohexylmethyl)-4-äthyl-2-hydroxy-5-hexenyl]-α-[(R)-α-(3,3-dimethyl-2-oxobutyl)hydrocinnamamido]imidazol-4-propionamid, (S)-α-[(S)-α-[(t-Butylsulfonyl)methyl]hydrocinnamamido]-N-[(1S,2S,4R)-1-(cyclohexylmethyl)-4-äthyl-2-hydroxy-5-hexenyl]-imidazol-5-propionamid, t-Butyl [(S)-α-[[(S)-1-[[(1S,2R oder 2S)-3-cyclohexyl-1-(cyclohexylmethyl)-2-hydroxypropyl]carbamoyl]-2-imidazol-4 yläthyl]carbamoyl]phenäthyl]carbamat, (2R oder S,3S)-3-(Boc-D-Pro- Phe-His-NH)-1,4-dicyclohexyl-2-butanol oder (S)-N-[(1S,2S)-1-(Cyclohexylmethyl)-4-äthyl-4-fluor-2-hydroxyhexyl]-α-[(R)-α-(3,3-dimethyl-2-oxobutyl)hydrocinnamamido]imidazol-4-propionamid.

15. Verbindungen der Formeln

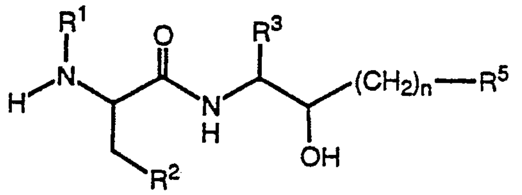

II

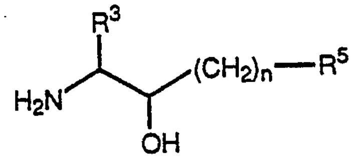

III

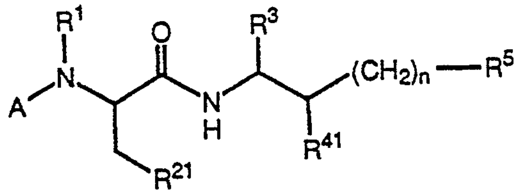

V

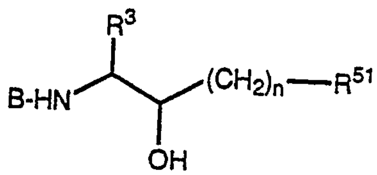

VI

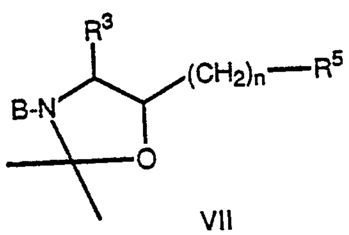

VII

und

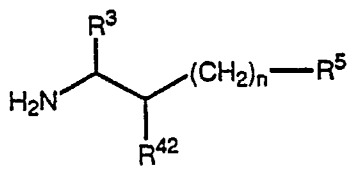

XIV

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^5$ Alkyl, Haloalkyl, Cycloalkyl, Alkenyl, Aryl oder Heteroaryl, n 0, 1, 2, 3, 4, 5 oder 6 und A eine der Gruppen

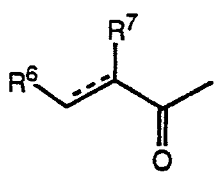

(a) und -Y-Z (b)

bedeuten, worin die gestrichelte Linie eine zusätzliche Bindung bedeuten kann, $R^6$ Phenyl, substituiertes Phenyl, Benzyl oder Naphthyl und $R^7$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes

Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin und Z Wasserstoff oder Acyl bedeuten, mit den Massgaben, dass

(i) falls n 2, 3, 4, 5 oder 6 bedeutet, $R^5$ nicht Alkyl, Cycloalkyl oder Aryl bedeutet,

(ii) falls n 1 und $R^5$ Alkyl bedeuten, das an die $CH_2$-Gruppe gebundene Kohlenstoffatom von Alkyl verzweigt ist,

(iii) falls n 0 und $R^5$ Alkyl bedeuten, das an das den Substituenten $R^4$ tragende Kohlenstoffatom gebundene Kohlenstoffatom von $R^5$ in α-Stellung keine Methylengruppe aufweist,

(iv) falls $R^2$ Imidazol-4-yl, $R^4$ Hydroxy und Y Phenylalanin bedeuten, $R^5$ nicht Alkyl bedeutet und

(v) falls $R^6$ Phenyl, Benzyl oder α-Naphthyl bedeutet, $R^7$ nicht Alkoxycarbonylamino oder Arylalkoxycarbonylamino bedeutet,

$R^{41}$ Hydroxy oder N-geschütztes Amino und $R^{21}$ Aethyl, Propyl, Isopropyl, Thiazol-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl bedeuten, mit der Massgabe, dass mindestens einer von $R^{41}$ und $R^{21}$ eine N-Schutzgruppe enthält, $R^{42}$ N-geschütztes Amino, $R^{51}$ Alkyl, Cycloalkyl, Alkenyl, Aryl oder Heteroaryl und B eine Aminoschutzgruppe bedeuten.

16. Aminosäurederivate gemäss einem der Ansprüche 1-14 zur Anwendung als therapeutische Wirkstoffe.

17. Aminosäurederivate gemäss einem der Ansprüche 1-14 zur Anwendung bei der Bekämpfung bzw. Verhütung von Bluthochdruck und Herzinsuffizienz.

18. Verfahren zur Herstellung einer Verbindung gemäss einem der Ansprüche 1-14, dadurch gekennzeichnet, dass man

a) zur Herstellung einer Verbindung der Formel I, worin $R^4$ Hydroxy bedeutet und die übrigen Symbole die in Anspruch 1 angegebene Bedeutung besitzen, eine Verbindung der allgemeinen Formel

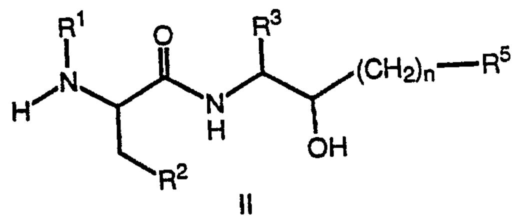

II

worin $R^1$, $R^2$, $R^3$, $R^5$ und n die in Anspruch 1 angegebene Bedeutung besitzen, mit einem die Gruppe

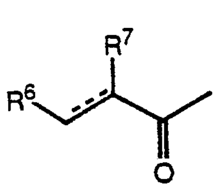

(a) oder -Y-Z (b)

worin $R^6$, $R^7$, Y, Z und die gestrichelte Linie die in Anspruch 1 angegebene Bedeutung besitzen, abgebenden Acylierungsmittel umsetzt, oder

b) zur Herstellung einer Verbindung der Formel I, worin $R^4$ Hydroxy bedeutet und die übrigen Symbole die in Anspruch 1 angegebene Bedeutung besitzen, eine Verbindung der allgemeinen Formel

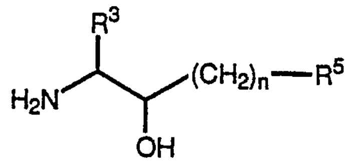

III

worin $R^3$, $R^5$ und n die in Anspruch 1 angegebene Bedeutung besitzen, mit einer Verbindung der allgemeinen Formel

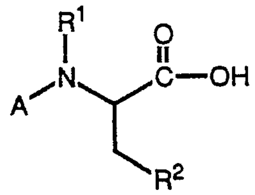

IV

worin $R^1$, $R^2$ und A die in Anspruch 1 angegebene Bedeutung besitzen, oder einem aktivierten Derivat davon umsetzt, oder

c) zur Herstellung einer Verbindung der Formel I, worin A die Gruppe (b), Z den einwertigen, über die Carboxylgruppe gebundenen Rest einer gegebenenfalls acylierten Aminosäure oder eines gegebenenfalls acylierten Dipeptids und $R^4$ Hydroxy bedeuten und die übrigen Symbole die in Anspruch 1 angegebene Bedeutung besitzen, eine Verbindung der Formel I, worin Z Wasserstoff bedeutet und die übrigen Symbole die in Anspruch 1 angegebene Bedeutung besitzen, mit einer gegebenenfalls acylierten Aminosäure oder einem gegebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeuten, aus einer entsprechenden Verbindung der Formel I, worin A eine N-geschützte Aminogruppe enthält, und/oder aus einer Verbindung der allgemeinen Formel

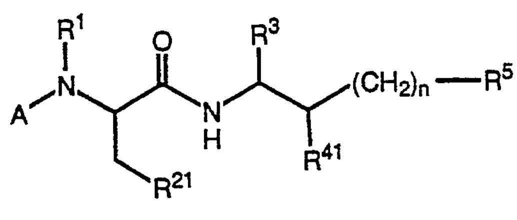

V

worin $R^{41}$ Hydroxy oder N-geschütztes Amino und $R^{21}$ Aethyl, Propyl, Isopropyl, Thiazol-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl bedeuten und die übrigen Symbole die in Anspruch 1 angegebene Bedeutung besitzen, mit der Massgabe, dass mindestens einer von $R^{41}$ und $R^{21}$ eine N-Schutzgruppe enthält, die N-Schutzgruppe(n) abspaltet, und

e) erwünschtenfalls ein Gemisch diastereomerer Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

19. Arzneimittel, enthaltend ein Aminosäurederivat gemäss einem der Ansprüche 1-14 und ein therapeutisch inertes Excipiens.

20. Mittel zur Bekämpfung bzw. Verhütung von Bluthochdruck und Herzinsuffizienz, enthaltend ein Aminosäurederivat gemäss einem der Ansprüche 1-14 und ein therapeutisch inertes Excipiens.

21. Verwendung eines Aminosäurederivates gemäss einem der Ansprüche 1-14 bei der Bekämpfung bzw. Verhütung von Krankheiten.

22. Verwendung eines Aminosäurederivates gemäss einem der Ansprüche 1-14 bei der Bekämpfung bzw. Verhütung von Bluthochdruck und Herzinsuffizienz.

23. Verwendung eines Aminosäurederivates gemäss einem der Ansprüche 1-14 zur Herstellung von Mitteln gegen Bluthochdruck und/oder Herzinsuffizienz.

Patentansprüche für folgenden Vertragsstaat: ES

1. Verfahren zur Herstellung von Aminosäurederivaten der allgemeinen Formel

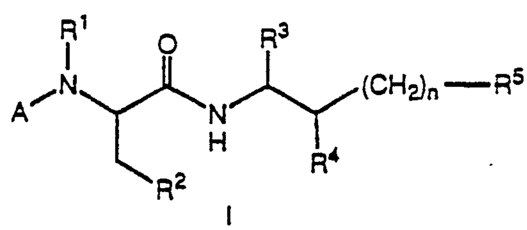

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Hydroxy oder Amino, $R^5$ Alkyl, Haloalkyl, Cycloalkyl, Alkenyl, Aryl oder Heteroaryl, n 0, 1, 2, 3, 4, 5 oder 6 und A eine der Gruppen

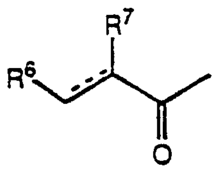

(a) und -Y-Z (b)

bedeuten, worin die gestrichelte Linie eine zusätzliche Bindung bedeuten kann, $R^6$ Phenyl, substituiertes Phenyl, Benzyl oder Naphthyl und $R^7$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxy phosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin und Z Wasserstoff oder Acyl bedeuten, mit den Massgaben, dass

(i) falls n 2, 3, 4, 5 oder 6 bedeutet, $R^5$ nicht Alkyl, Cycloalkyl oder Aryl bedeutet,

(ii) falls n 1 und $R^5$ Alkyl bedeuten, das an die $CH_2$-Gruppe gebundene Kohlenstoffatom von Alkyl verzweigt ist,

(iii) falls n 0 und $R^5$ Alkyl bedeuten, das an das den Substituenten $R^4$ tragende Kohlenstoffatom gebundene Kohlenstoffatom von $R^5$ in α-Stellung keine Methylengruppe aufweist,

(iv) falls $R^2$ Imidazol-4-yl, $R^4$ Hydroxy und Y Phenylalanin bedeuten, $R^5$ nicht Alkyl bedeutet und

(v) falls $R^6$ Phenyl, Benzyl oder α-Naphthyl bedeutet, $R^7$ nicht Alkoxycarbonylamino oder Arylalkoxycarbonylamino bedeutet,

in Form von optisch reinen Diastereomeren, Diastereomerengemischen, diastereomeren Racematen oder Gemischen von diastereomeren Racematen sowie von pharmazeutisch verwendbaren Salzen dieser Verbindungen, dadurch gekennzeichnet, dass man

a) zur Herstellung einer Verbindung der Formel I, worin $R^4$ Hydroxy bedeutet und die übrigen Symbole die oben angegebene Bedeutung besitzen, eine Verbindung der allgemeinen Formel

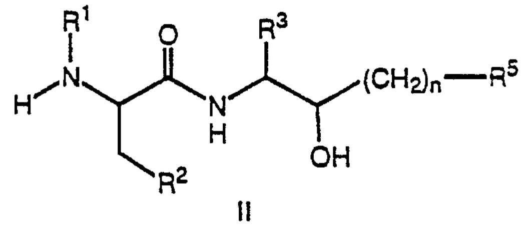

II

worin $R^1$, $R^2$, $R^3$, $R^5$ und n die oben angegebene Bedeutung besitzen, mit einem die Gruppe

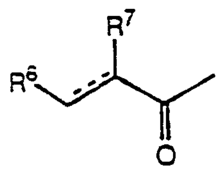 (a) oder -Y-Z (b)

worin $R^6$, $R^7$, Y, Z und die gestrichelte Linie die oben angegebene Bedeutung besitzen, abgebenden Acylierungsmittel umsetzt, oder

b) zur Herstellung einer Verbindung der Formel I, worin $R^4$ Hydroxy bedeutet und die übrigen Symbole die oben angegebene Bedeutung besitzen, eine Verbindung der allgemeinen Formel

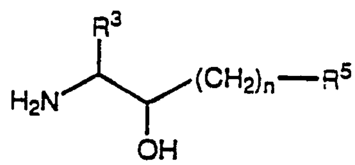

III

worin $R^3$, $R^5$ und n die oben angegebene Bedeutung besitzen, mit einer Verbindung der allgemeinen Formel

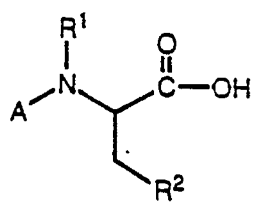

IV

worin $R^1$, $R^2$ und A die oben angegebene Bedeutung besitzen, oder einem aktivierten Derivat davon umsetzt, oder

c) zur Herstellung einer Verbindung der Formel I, worin A die Gruppe (b), Z den einwertigen, über die Carboxylgruppe gebundenen Rest einer gegebenenfalls acylierten Aminosäure oder eines gegebenenfalls acylierten Dipeptids und $R^4$ Hydroxy bedeuten und die übrigen Symbole die oben angegebene Bedeutung besitzen, eine Verbindung der Formel I, worin Z Wasserstoff bedeutet und die übrigen Symbole die oben angegebene Bedeutung besitzen, mit einer gegebenenfalls acylierten Aminosäure oder einem gebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeuten, aus einer entsprechenden Verbindung der Formel I, worin A eine N-geschützte Aminogruppe enthält, und/oder aus einer Verbindung der allgemeinen Formel

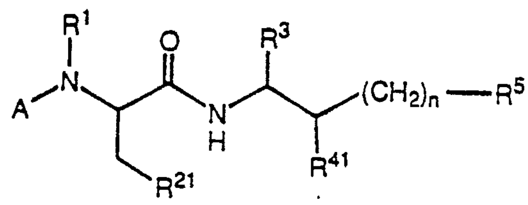

V

worin $R^{41}$ Hydroxy oder N-geschütztes Amino und $R^{21}$ Aethyl, Propyl, Isopropyl, Thiazol-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl bedeuten und die übrigen Symbole die in Anspruch 1 angegebene Bedeutung besitzen, mit der Massgabe, dass mindestens einer von $R^{41}$ und $R^{21}$ eine N-Schutzgruppe enthält, die N-Schutzgruppe(n) abspaltet, und

e) erwünschtenfalls ein Gemisch diastereomerer Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

2. Verfahren gemäss Anspruch 1, worin $R^1$ Wasserstoff bedeutet.

3. Verfahren gemäss Anspruch 1 oder 2, worin $R^2$ Imidazol-2-yl, Imidazol-4-yl oder Thiazol-4-yl, insbesondere Imidazol-4-yl, bedeutet.

4. Verfahren gemäss einem der Ansprüche 1-3, worin $R^3$ Cyclohexylmethyl bedeutet.

5. Verfahren gemäss einem der Ansprüche 1-4, worin $R^4$ Hydroxy bedeutet.

6. Verfahren gemäss einem der Ansprüche 1-5, worin $R^5$ Alkyl, Haloalkyl, Cycloalkyl oder Alkenyl, insbesondere 3-Pentyl, Fluoralkyl, Cyclohexyl oder 3-Pentenyl, bedeutet.

7. Verfahren gemäss einem der Ansprüch 1-6, worin n 0 oder 1, insbesondere 1, ist.

8. Verfahren gemäss einem der Ansprüche 1-7, worin A die Gruppe (a) bedeutet.

9. Verfahren gemäss Anspruch 8, worin $R^6$ Phenyl oder substituiertes Phenyl, insbesondere Phenyl, bedeutet.

10. Verfahren gemäss Anspruch 8 oder 9, worin $R^7$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, insbesondere Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeutet.

11. Verfahren gemäss einem der Ansprüche 1-7, worin A die Gruppe (b) und Y den N-terminal mit Z verbundenen, bivalenten Rest von Phenylalanin bedeutet.

12. Verfahren gemäss Anspruch 11, worin Z die Gruppe $R^a$-O-CO- oder den Rest einer durch diese Gruppe acylierten α-Aminosäure, insbesondere Prolin, bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, inbesondere einen gesättigten aliphatischen Kohlenwasserstoffrest mit bis zu 6 Kohlenstoffatomen, bedeutet.

13. Verfahren gemäss Anspruch 1, worin $R^1$ Wasserstoff, $R^2$ Imidazol-4-yl, $R^3$ Cyclohexylmethyl, $R^4$ Hydroxy, $R^5$ 3-Pentyl, Fluoralkyl, Cyclohexyl oder 3-Pentenyl, n 1, $R^6$ Phenyl, $R^7$ $C_1$-$C_4$-Alkylcarbonylmethyl oder $C_1$-$C_4$-Alkylsulfonylmethyl, Y den N-terminal mit Z verbundenen, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen bedeutet.

14. Verfahren gemäss Anspruch 1, dadurch gekennzeichnet, dass man t-Butyl (R)-2-[[(S)-α-[[(S)-1-[[(1S,2S,4R)-1-(cyclohexylmethyl)-2-hydroxy-4-äthyl-5-hexenyl]carbamoyl]-2-imidazol-4-yläthyl]carbamoyl]-phenäthyl]carbamoyl]-1-pyrrolidincarboxylat herstellt.

15. Verfahren gemäss Anspruch 1, dadurch gekennzeichnet, dass man t-Butyl (R)-2-[[(S)-α-[[(S)-1-[[(1S,2S)-1-(cyclohexylmethyl)-2-hydroxy-4-äthylhexyl]carbamoyl]-2-imidazol-4-yläthyl]carbamoyl]phenäthyl]-carbamoyl]-1-pyrrolidincarboxylat herstellt.

16. Verfahren gemäss Anspruch 1, dadurch gekennzeichnet, dass man (S)-N-[(1S,2S,4R)-1-(Cyclohexylmethyl)-4-äthyl-2-hydroxy-5-hexenyl]-α-[(R)-α-(3,3-dimethyl-2-oxobutyl)hydrocinnamamido]-imidazol-4-propionamid herstellt.

17. Verfahren gemäss Anspruch 1, dadurch gekennzeichnet, dass man (S)-α-[(S)-α-[(t-Butylsulfonyl)-

methyl]hydrocinnamamido]-N-[(1S,2S,4R)-1-(cyclohexylmethyl)-4-äthyl-2-hydroxy-5-hexenyl]imidazol-5-propionamid herstellt.

18. Verfahren gemäss Anspruch 1, dadurch gekennzeichnet, dass man t-Butyl [(S)-α-[[(S)-1-[[(1S,2R oder 2S)-3-cyclohexyl-1-(cyclohexylmethyl)-2-hydroxypropyl]carbamoyl]-2-imidazol-4-yläthyl]-carbamoyl]-phenäthyl]carbamat herstellt.

19. Verfahren gemäss Anspruch 1, dadurch gekennzeichnet, dass man (2R oder S,3S)-3-(Boc-D-Pro-Phe-His-NH)-1,4-dicyclohexyl-2-butanol herstellt.

20. Verfahren gemäss Anspruch 1, dadurch gekennzeichnet, dass man (S)-N-[(1S,2S)-1-(Cyclohexylmethyl)-4-äthyl-4 fluor-2-hydroxyhexyl]-α-[(R)-α-(3,3-dimethyl-2-oxobutyl)hydrocinnamamido]-imidazol-4-propionamid herstellt.

21. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

22. Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

Patentansprüche für folgenden Vertragsstaat: GR

1. Verfahren zur Herstellung von Aminosäurederivaten der allgemeinen Formel

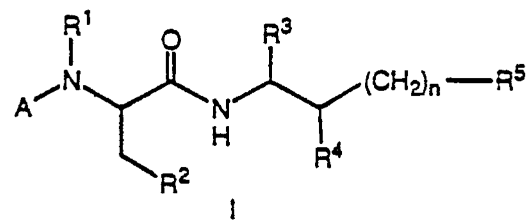

I

worin $R^1$ Wasserstoff oder Methyl, $R^2$ Aethyl, Propyl, Isopropyl, Imidazol-2-yl, Imidazol-4 yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Hydroxy oder Amino, $R^5$ Alkyl, Haloalkyl, Cycloalkyl, Alkenyl, Aryl oder Heteroaryl, n 0, 1, 2, 3, 4, 5 oder 6 und A eine der Gruppen

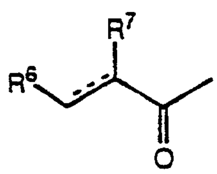

(a) und -Y-Z (b)

bedeuten, worin die gestrichelte Linie eine zusätzliche Bindung bedeuten kann, $R^6$ Phenyl, substituiertes Phenyl, Benzyl oder Naphthyl und $R^7$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxy phosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin und Z Wasserstoff oder Acyl bedeuten,

mit den Massgaben, dass

(i) falls n 2, 3, 4, 5 oder 6 bedeutet, $R^5$ nicht Alkyl, Cycloalkyl oder Aryl bedeutet,

(ii) falls n 1 und $R^5$ Alkyl bedeuten, das an die $CH_2$-Gruppe gebundene Kohlenstoffatom von Alkyl verzweigt ist,

(iii) falls n 0 und $R^5$ Alkyl bedeuten, das an das den Substituenten $R^4$ tragende Kohlenstoffatom gebundene Kohlenstoffatom von $R^5$ in α-Stellung keine Methylengruppe aufweist,

(iv) falls $R^2$ Imidazol-4-yl, $R^4$ Hydroxy und Y Phenylalanin bedeuten, $R^5$ nicht Alkyl bedeutet und

(v) falls $R^6$ Phenyl, Benzyl oder α-Naphthyl bedeutet, $R^7$ nicht Alkoxycarbonylamino oder Arylalkoxycarbonylamino bedeutet,

in Form von optisch reinen Diastereomeren, Diastereomerengemischen, diastereomeren Racematen oder Gemischen von diastereomeren Racematen sowie von pharmazeutisch verwendbaren Salzen dieser Verbindungen, dadurch gekennzeichnet, dass man

a) zur Herstellung einer Verbindung der Formel I, worin $R^4$ Hydroxy bedeutet und die übrigen Symbole die oben angegebene Bedeutung besitzen, eine Verbindung der allgemeinen Formel

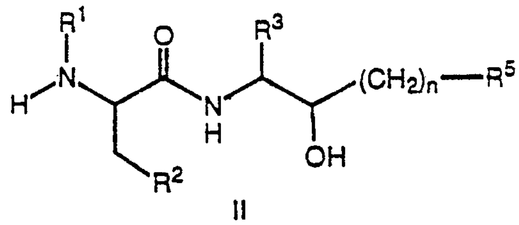

II

worin $R^1$, $R^2$, $R^3$, $R^5$ und n die oben angegebene Bedeutung besitzen,
mit einem die Gruppe

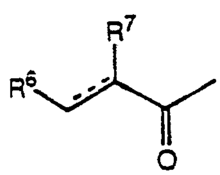

(a) oder -Y-Z (b)

worin $R^6$, $R^7$, Y, Z und die gestrichelte Linie die oben angegebene Bedeutung besitzen,
abgebenden Acylierungsmittel umsetzt, oder

b) zur Herstellung einer Verbindung der Formel I, worin $R^4$ Hydroxy bedeutet und die übrigen Symbole die oben angegebene Bedeutung besitzen, eine Verbindung der allgemeinen Formel

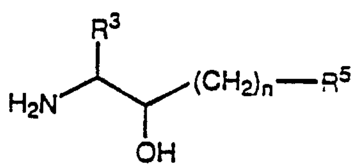

III

worin $R^3$, $R^5$ und n die oben angegebene Bedeutung besitzen,
mit einer Verbindung der allgemeinen Formel

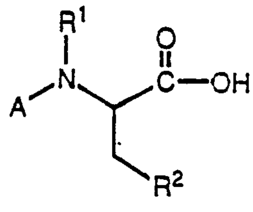

IV

worin $R^1$, $R^2$ und A die oben angegebene Bedeutung besitzen, oder einem aktivierten Derivat davon umsetzt, oder

c) zur Herstellung einer Verbindung der Formel I, worin A die Gruppe (b), Z den einwertigen, über die Carboxylgruppe gebundenen Rest einer gegebenenfalls acylierten Aminosäure oder eines gegebenenfalls acylierten Dipeptids und $R^4$ Hydroxy bedeuten und die übrigen Symbole die oben angegebene Bedeutung besitzen, eine Verbindung der Formel I, worin Z Wasserstoff bedeutet und die übrigen Symbole die oben angegebene Bedeutung besitzen, mit einer gegebenenfalls acylierten Aminosäure oder einem gegebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeuten, aus einer entsprechenden Verbindung der Formel I, worin A eine N-geschützte Aminogruppe enthält, und/oder aus einer Verbindung der allgemeinen Formel

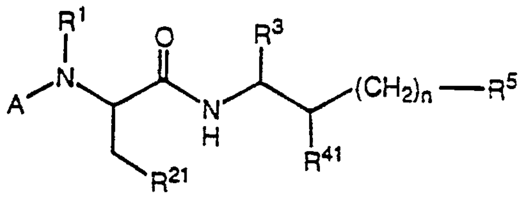

V

worin $R^{41}$ Hydroxy oder N-geschütztes Amino und $R^{21}$ Aethyl, Propyl, Isopropyl, Thiazol-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl bedeuten und die übrigen Symbole die in Anspruch 1 angegebene Bedeutung besitzen, mit der Massgabe, dass mindestens einer von $R^{41}$ und $R^{21}$ eine N-Schutzgruppe enthält, die N-Schutzgruppe(n) abspaltet, und

e) erwünschtenfalls ein Gemisch diastereomere, Racemate in die diastereomeren Racemate oder optisch reinen Diastereomeren auftrennt, und/oder

f) erwünschtenfalls ein Diastereomerengemisch in die optisch reinen Diastereomeren auftrennt, und/oder

g) erwünschtenfalls eine erhaltene Verbindung in ein pharmazeutisch verwendbares Salz überführt.

2. Verfahren gemäss Anspruch 1, worin $R^1$ Wasserstoff bedeutet.

3. Verfahren gemäss Anspruch 1 oder 2, worin $R^2$ Imidazol-2-yl, Imidazol-4-yl oder Thiazol-4-yl, insbesondere Imidazol-4-yl, bedeutet.

4. Verfahren gemäss einem der Ansprüche 1-3, worin $R^3$ Cyclohexylmethyl bedeutet.

5. Verfahren gemäss einem der Ansprüche 1-4, worin $R^4$ Hydroxy bedeutet.

6. Verfahren gemäss einem der Ansprüche 1-5, worin $R^5$ Alkyl, Haloalkyl, Cycloalkyl oder Alkenyl, insbesondere 3-Pentyl, Fluoralkyl, Cyclohexyl oder 3-Pentenyl, bedeutet.

7. Verfahren gemäss einem der Ansprüch 1-6, worin n 0 oder 1, insbesondere 1, ist.

8. Verfahren gemäss einem der Ansprüche 1-7, worin A die Gruppe (a) bedeutet.

9. Verfahren gemäss Anspruch 8, worin $R^6$ Phenyl oder substituiertes Phenyl, insbesondere Phenyl, bedeutet.

10. Verfahren gemäss Anspruch 8 oder 9, worin $R^7$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, insbesondere Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeutet.

11. Verfahren gemäss einem der Ansprüche 1-7, worin A die Gruppe (b) und Y den N-terminal mit Z verbundenen, bivalenten Rest von Phenylalanin bedeutet.

12. Verfahren gemäss Anspruch 11, worin Z die Gruppe $R^a$-O-CO- oder den Rest einer durch diese Gruppe acylierten α-Aminosäure, insbesondere Prolin, bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, inbesondere einen gesättigten aliphatischen Kohlenwasserstoffrest mit bis zu 6 Kohlenstoffatomen, bedeutet.

13. Verfahren gemäss Anspruch 1, worin $R^1$ Wasserstoff, $R^2$ Imidazol-4-yl, $R^3$ Cyclohexylmethyl, $R^4$ Hydroxy, $R^5$ 3-Pentyl, Fluoralkyl, Cyclohexyl oder 3-Pentenyl, n 1, $R^6$ Phenyl, $R^7$ $C_1$-$C_4$-Alkylcarbonylmethyl oder $C_1$-$C_4$-Alkylsulfonylmethyl, Y den N-terminal mit Z verbundenen, bivalenten Rest von Phenylalanin und Z die Gruppe $R^a$-O-CO- oder den durch diese Gruppe acylierten Rest von Prolin bedeuten, worin $R^a$ einen gesättigten, aliphatischen Kohlenwasserstoffrest mit bis zu 6 Kohlenstoffatomen bedeutet.

14. Verfahren gemäss Anspruch 1, dadurch gekennzeichnet, dass man t-Butyl (R)-2-[[(S)-α-[[(S)-1-[[(1S,2S,4R)-1-(cyclohexylmethyl)-2-hydroxy-4-äthyl-5-hexenyl]carbamoyl]-2-imidazol-4-yläthyl]carbamoyl]-phenäthyl]carbamoyl]-1-pyrrolidincarboxylat herstellt.

15. Verfahren gemäss Anspruch 1, dadurch gekennzeichnet, dass man t-Butyl (R)-2-[[(S)-α-[[(S)-1-[[(1S,2S)-1-(cyclohexylmethyl)-2-hydroxy-4-äthylhexyl]carbamoyl]-2-imidazol-4-yläthyl]carbamoyl]phenäthyl]-carbamoyl]-1-pyrrolidincarboxylat herstellt.

16. Verfahren gemäss Anspruch 1, dadurch gekennzeichnet, dass man (S)-N-[(1S,2S,4R)-1-(Cyclohexylmethyl)-4-äthyl-2-hydroxy-5-hexenyl]-α-[(R)-α-(3,3-dimethyl-2-oxobutyl)hydrocinnamamido]-imidazol-4-propionamid herstellt.

17. Verfahren gemäss Anspruch 1, dadurch gekennzeichnet, dass man (S)-α-[(S)-α-[(t-Butylsulfonyl)-methyl]hydrocinnamamido]-N-[(1S,2S,4R)-1-(cyclohexylmethyl)-4-äthyl-2-hydroxy-5-hexenyl]imidazol-5-propionamid herstellt.

18. Verfahren gemäss Anspruch 1, dadurch gekennzeichnet, dass man t-Butyl [(S)-α-[[(S)-1-[[(1S,2R oder 2S)-3-cyclohexyl-1-(cyclohexylmethyl)-2-hydroxypropyl]carbamoyl]-2-imidazol-4-yläthyl]-carbamoyl]-phenäthyl]carbamat herstellt.

19. Verfahren gemäss Anspruch 1, dadurch gekennzeichnet, dass man (2R oder S,3S)-3-(Boc-D-Pro-Phe-His-NH)-1,4-dicyclohexyl-2-butanol herstellt.

20. Verfahren gemäss Anspruch 1, dadurch gekennzeichnet, dass man (S)-N-[(1S,2S)-1-(Cyclohexylmethyl)-4-äthyl-4-fluor-2-hydroxyhexyl]-α-[(R)-α-(3,3-dimethyl-2-oxobutyl)hydrocinnamamido]-imidazol-4-propionamid herstellt.

21. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

22. Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

23. Verbindungen der Formeln

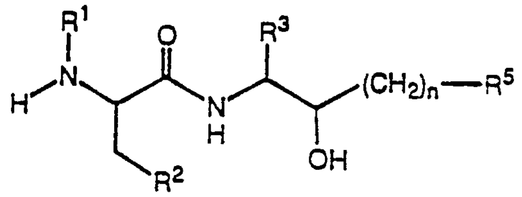

II

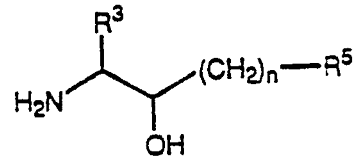

III

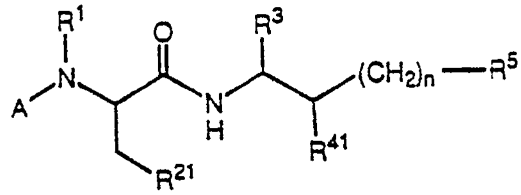

V

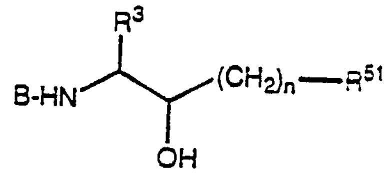

VI

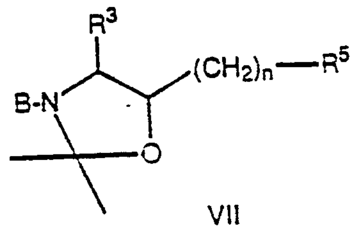

VII

und

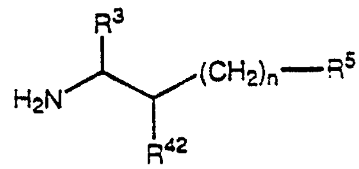

XIV

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^5$ Alkyl, Haloalkyl, Cycloalkyl, Alkenyl, Aryl oder Heteroaryl, n 0, 1, 2, 3, 4, 5 oder 6 und A eine der Gruppen

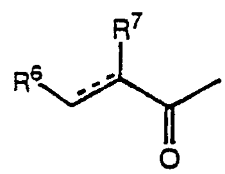

(a) und -Y-Z (b)

bedeuten, worin die gestrichelte Linie eine zusätzliche Bindung bedeuten kann, $R^6$ Phenyl, substituiertes Phenyl, Benzyl oder Naphthyl und $R^7$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin und Z Wasserstoff oder Acyl bedeuten, mit den Massgaben, dass

(i) falls n 2, 3, 4, 5 oder 6 bedeutet, $R^5$ nicht Alkyl, Cycloalkyl oder Aryl bedeutet,

(ii) falls n 1 und $R^5$ Alkyl bedeuten, das an die $CH_2$-Gruppe gebundene Kohlenstoffatom von Alkyl verzweigt ist,

(iii) falls n 0 und $R^5$ Alkyl bedeuten, das an das den Substituenten $R^4$ tragende Kohlenstoffatom gebundene Kohlenstoffatom von $R^5$ in α-Stellung keine Methylengruppe aufweist,

(iv) falls $R^2$ Imidazol-4-yl, $R^4$ Hydroxy und Y Phenylalanin bedeuten, $R^5$ nicht Alkyl bedeutet und

(v) falls $R^6$ Phenyl, Benzyl oder α-Naphthyl bedeutet, $R^7$ nicht Alkoxycarbonylamino oder Arylalkoxycarbo-

nylamino bedeutet,
$R^{41}$ Hydroxy oder N-geschütztes Amino und $R^{21}$ Aethyl, Propyl, Isopropyl, Thiazol-4-yl, Thien-2-yl, Aethoxycarbonyl, t-Butylcarbonylmethyl, Benzyloxycarbonylmethyl, t-Butoxy oder gegebenenfalls N-geschütztes Imidazol-2-yl, Imidazol-4-yl oder Pyrazol-3-yl bedeuten, mit der Massgabe, dass mindestens einer von $R^{41}$ und $R^{21}$ eine N-Schutzgruppe enthält, $R^{42}$ N-geschütztes Amino, $R^{51}$ Alkyl, Cycloalkyl, Alkenyl, Aryl oder Heteroaryl und B eine Aminoschutzgruppe bedeuten.